# EUROPÄISCHE PATENTSCHRIFT

(11) **EP 1 019 543 B1**
(45) Veröffentlichungstag und Bekanntmachung des Hinweises auf die Patenterteilung: **02.11.2006**
(21) Anmeldenummer: 98951474.0
(22) Anmeldetag: 26.09.1998
(51) Int. Cl.: C12Q 1/68, C12N 15/62

(54) **VERFAHREN ZUM IDENTIFIZIEREN EINER NUKLEINSÄURE**
METHOD FOR IDENTIFYING A NUCLEIC ACID
PROCEDE D'IDENTIFICATION D'UN ACIDE NUCLEIQUE

(30) Priorität: 27.09.1997 EP 97116841; 29.10.1997 EP 97118755
(43) Veröffentlichungstag der Anmeldung: 19.07.2000
(73) Patentinhaber: Evotec AG, 22525 Hamburg (DE)
(72) Erfinder: STRAUSS, Andreas, D-22525 Hamburg (DE); THUMM, Günther, D-22525 Hamburg (DE); POHLNER, Johannes, D-22525 Hamburg (DE); GÖTZ, Friedrich, D-22525 Hamburg (DE)
(74) Vertreter: Meyers, Hans-Wilhelm
(86) Internationale Anmeldenummer: PCT/EP1998/006136
(87) Internationale Veröffentlichungsnummer: WO 1999/016900

(56) Entgegenhaltungen:
- WO-A-96/34976
- WO-A-97/08553
- US-A- 5 616 686
- HUNG TON-THAT: "Anchor structure of Staphylococcal surface proteins" THE JOURNAL OF BIOLOGICAL CHEMISTRY, Bd. 272, Nr. 35, August 1997, Seiten 22285-22292, XP002059728
- SCHNEEWIND O ET AL: "SORTING OF PROTEIN A TO THE STAPHYLOCOCCAL CELL WALL" CELL, Bd. 70, 24. Juli 1992, Seiten 267-281, XP002017666 in der Anmeldung erwähnt
- SCHNEEWIND, O. ET AL.: "Structure of the cell wall anchor of surface proteins in Staphylococcus aureus" SCIENCE, Bd. 268, 7. April 1995, Seiten 103-106, XP002059729 in der Anmeldung erwähnt
- SAMUELSON P ET AL: "CELL SURFACE DISPLAY OF RECOMBINANT PROTEINS ON STAPHYLOCOCCUS CARNOSUS" JOURNAL OF BACTERIOLOGY, Bd. 177, Nr. 6, März 1995, Seiten 1470-1476, XP000612670
- ROUCH, D.A. ET AL.: "trimethoprim resistance transposon Tn4003 from staphylococcus aureus encodes genes for a dihydrofolate reductase and thymidylate synthetase flanked by three copies of IS257." MOLECULAR MICROBIOLOGY, Bd. 3, Nr. 2, 1989, Seiten 161-175, XP002059730
- MURAKAMI, Y. ET AL.: "Role of the charged tail in localasation of a surface protein antigen in Streptococcus mutans" INFECT. IMMUN., Bd. 65, Nr. 4, April 1997, Seiten 1531-1523, XP002059731

### SEQUENZPROTOKOLL

(1) ALLGEMEINE ANGABEN:
   (i) ANMELDER:
      (A) NAME: Evotec BioSystems GmbH
      (B) STRASSE: Grandweg 64
      (C) ORT: Hamburg
      (E) LAND: Deutschland
      (F) POSTLEITZAHL: D-22529
   (ii) BEZEICHNUNG DER ERFINDUNG: Verfahren zum Identifizieren einer Nukleinsäure
   (iii) ANZAHL DER SEQUENZEN: 17
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 604 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 1:
   ACCCATGTTG GAAAGAATCA TATTCTCCAT GTAAATCACT GACAAAGTGT TCAGTGCCTT    60
   TTGTTAGATT CTAGACTGAT GATTTCTGTA GCGAGTTGTT CTTCGTGATT AAATTTTTCT   120
   GCAAGTAAGT CAAAGTATTT ATGTTTAAGT TCACGATCTG TGGTCTTCAT TTCTTCCTCC   180
   TGTGCTTCTA TATTAATAGC GCTTACATTT GATTATTGTA CTTTTTTATA AAATTTTCAA   240
   TGGTAGCGGT TAAATTGTTG CAAATTAGTT GCATTAAAAG TAAACGATTT CAAATTTAGG   300
   TGAGAATTAG GTAGTATTTA CTATAATCTG AGACAATCTC CTAGAAACAT GTGATATTAT   360
   TATTTTTGAA TACAATATTT TWACATCAGG AGGCATTATG ACATCCCTAT TTTCTGTAGA   420
   GCAATTGAAC AAATCTTTCA AAGACAGCAC TTTTAAAATA AATAATGTGT CATTTGAAGT   480
   GCATGAAGGT GAAATTGTAG CGTTTTTCGG CCAGAACGGC TCTGGCAAAT CTACTTTGAT   540
   TCGTATGATT GTGGGTGATT ATCCCTTGTT CCAGGGAAAT TGTTTTTTTT KGTGNNGCGG   600
   TGGG                                                                604
(2) ANGABEN ZU SEQ ID NO: 2:
   (i) SEQUENZKENNZEICHEN:
      (A) LÄNGE: 295 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 2:
   CTAAAAAGGA GGATGCCAAC TATGAATAAA TTTGACAAAG CATATGAGAT TTGTGCAAAG    60
   AGATTCTTGA AATCGGAAAT GAACGTGACG ATCGTACACA TACAGGTACA ATTTCAAAGT   120
   TCGGCCACCA ATTACGGTTT GATTTATCTG AAGGATTTCC ATTACTTACT ACAAAAAAAG   180
   TTTCGTTTAA ATTATTAGCT ACTGAACTCG TTTGGTTCAT AAAAGGTGAT ACTAAGATCA   240
   AGTACTTACT TCAATATAGT AATAATATCC GGAATGAGTG GGCATTTGAA AAATA        295
   (i) SEQUENZKENNZEICHEN:
      (A) LÄNGE: 429 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 3:
(2) ANGABEN ZU SEQ ID NO: 4:
   (i) SEQUENZKENNZEICHEN:
      (A) LÄNGE: 748 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 4:
   KCGGGAGCCT AATACTAAAA ATAATGAATT CAATGTCGAA AATTAAACTG AATGCATTAC    60
   GTGAGCAACG TCATATATTA AATGAATTGG TAGATGAAGG AGAAGTAAGT GAAAAAACGG   120
   CACTGAAAGT AAGAGAAGAG GTTAATTATG ATGAAATGAT TTTAGTAGAG TCGCTTAACT   180
   TAATATATTA AATAAGCTGT AAAACAGATG CTTAGCGACG ATTGTTTTGC AGCTTTTTTA   240
   TGTAAAAMAG GAGCCAGTCT TGGAGGGGAC TGGCTCTTTG AAAAGCGATA TCATCGATTA   300
   CCGCTTTTTC CTTAGGGGTT TTTGTGGTCT CTAGGGAGGA TTACGAGCAC TGCTTATTAC   360
   TTATGTTTAG GATAGATTAT TTATGGGAGA TGACGGTATT TGAATTAGGA GGTGAACCGG   420
   CATCTTAAGG ATAATGTGTT ATTTCATACT GTTGTGGACA GTTTCAATAT TAGATTCCMC   480
   CATTTTGTAM TAGGAATCGC CCTTACTGCC TTTTTTGCCA ATAGAGTCTG TATAAACTTC   540
   TCCMGAAATC GGTACACCTG TTTCTTCACY TAAACYYTGC RGGACTTTTG TGGGGCCCCA   600
   CCTGGGGTYT CGCGCCAAAA AGGAGGGTTT TAAGGGAAGG GATTTTACAA ATTGAANCGC   660
   CTGTTCAACT TGTTCGGTGT CCCCGTTTTT CCGAATTGAG TTCCACAATA TAGCGGGTGT   720
   AATGCCCTAT TTGTTTAGAG AAATATTT                                       748
   (i) SEQUENZKENNZEICHEN:
      (A) LÄNGE: 572 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 5:
(2) ANGABEN ZU SEQ ID NO: 6:
   (i) SEQUENZKENNZEICHEN:
      (A) LÄNGE: 690 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 6:
   CTAAAAAACA ATAAAADGTG CAGTGTAGAA AATTAATTCT AGCGACTGAC TTTTTTATTA     60
   TGATGCTTTA TCATTGAAAA TTGCATTAAG GTTAAAGAAT TTAGTTAATG CATTTCCGCT    120
   TTGTTGATTA TTATTGCCTT GGTATTATTA CCTTGTTGAT TATTATTGCC TTGGAGTTAT    180
   TACCTTGTTG ATTGCTTTGT CCAGAACTAT TATTTGAACC ACTGTTACTA CTGCTGCTGC    240
   CACCATTTAC AGTACCGTTA CTATTAACTT TTCTATTAGT AGTATCGTTG TCTGGGTGTC    300
   CAGCTACAGA TAAATCTTCT TTGCTTGAAC CATCTACAGA AGAAGGTTTT TTGAAGTCTG    360
   CACCGTCACG AGGACTAATG TCTGACATTA CAATCTTCAA GTAAATATTG TGGATAATCT    420
   TGTTCACTAT GACCAAACAA ATGAATTTTC ACCATAATTG TTTTACTTTA TTGAAAGCCA    480
   TCCAAAACTG ACATTGAATA TTTAGGTGTG AAACAATTAA TCCAACATCT TTGGCTGCCG    540
   TCATCGGTAA GTTGTATTGT TGAAAATGTT TCACTACCGT AAGTAACCTG TCCCAGTTTT    600
   TGCTGCTAGA TTAACAACCT GAAACGCCGT GTCCCAAAAA AGCAGAACCC CAAGCTTCAA    660
   AAGTACCTTT TAAAGAAATT CCTGAAAGGC                                     690
(2) ANGABEN ZU SEQ ID NO: 7:
   (i) SEQUENZKENNZEICHEN:
      (A) LÄNGE: 459 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 7:
   CATAATAAGG TCGATACTCA GGGTTTTGTA TTAACTCTTT TTGTGAATGT CCTCAAATTT     60
   TCCTAGTGAA CGTTCTCTTA ACAATGCTGT AATAGTAACA GGAATAGTGT TATCAAAACC    120
   ATTTTCATAG GTCTGTACAG TCCTCTTTAA ATCTGAAACA TAAATATGGT CAATAGGAAT    180
   ATCTGAAAAG TATTCTTTCA ATTTTTTTGC TGACTTGATA CCTGTTTCAG TCAAAGCAAC 240
   ATTTAGTTGA CCGCAAAAGT AATCTTGCCC GTGTTTATTA TCATAATTTG CTGTCGATTC 300
   TCCGTCTCGA ATTAAATAAA TCTCCAAGTT GTCTCACTCC AATTATTAAC TTACTTTCAG 360
   GCCAGTTACT TTACTGATAT CTTTATWWAG ATAATAAAGT TATAGACACC ACTAAATATT 420
   TTATTCAACA GTATGATAAC ACGGTTTTWA TCAATATAT 459
(2) ANGABEN ZU SEQ ID NO: 8:
   (i) SEQUENZKENNZEICHEN:
      (A) LÄNGE: 800 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 8:
   CCAGATTAGA AGTATATACC AGAAGAAGCT GAGAAGCACC TGGTACTCTT GTTGGGTATG 60
   CCGATAATTA CATGAAAGTT GAATTTGAAG GTGACGATTC ATTAATAGGA GAGTTGGTTC 120
   GTGTTAAAAT TACACAAGCT GGATATCCAT TGAATCAAGG ACATGTAGTT MKAGTTATTG 180
   ACCATGCATC TAACCGTAGT GAATTTACTG CTATTATTTA AGATTCAAAA AATTAATTAT 240
   TCTAATTGAC CCCAACTATC ATATATATTA TACTTATGAA TGCGTCATAT TTAGTTATGC 300
   TATGTATATG AAAATAAAGT TCCGTTGATA TTTGGAGGGA GGGAAATACA GATGTCTAAA 360
   ACMGTAGTCC GTAAAAACGA ATCACTTGAA GATGCTTTAC GCCGTTTCAA ACGCTCAGTT 420
   TCAAAAAGCG GTACAATTCA AGAAGTACGT AAACGTGAAT TTTACGAAAA ACCAAGTGTT 480
   AAACGTAAAA AGAAATCAGA AGCTGCACGT AAACGTAAAT TTAAATAATT GATATGTCTG 540
   TTGACTCCCT CAACAACAAA TATGAATTAT ATAAATGCCG TTTTTCGAAG GTCATATATA 600
   TTACCAATTC AGGTAGTATT TATGGCCTTT TTTCTTCTCA TTTTAAAATC AAAATATATT 660
   TTCTATAAAT TCACTCTTTT ATAACTATAA TTCCTTTAAG TAGTCMTGTA TAATGAAATG 720
   AGAGCGAGGT GAATTTTTGT CTCATCCCAC CTGGGGATTT TTACCTAATA ATTTCCGTGT 780
   TTCAGACTAG TCNAAGCCCC 800
(2) ANGABEN ZU SEQ ID NO: 9:
   (i) SEQUENZKENNZEICHEN:
      (A) LÄNGE: 684 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 9:
   CTATTAGTAA AAACAACAGA AAGATAGATT GTATTAGTAA AAACAACAGA AAGATAGAGA 60
   TATGGAGCAA TTATTTTTCT TTTCCTTATT TTAGTTTACA TAATATATAT TATAGGAAGT 120
   TATATATAGA ACGAGAGAGA GTGCTTTGCA TATCCCTATT TATATGTGTA TGTCTTATTT 180
   TGTATTGTTG TTCAGCTTTT ATTTAACTAC ATATTAGCGA ACTCTATTTC TTGTTACTCA 240
   TGTGTTTGTA ATTACATAGC CATGAGGTTT ACGTTTCGAC TATATCGATA TGGAATCGCA 300
   ACTATAATGG TATATAACGA TATATGTAAA TCAGTTATAA AACGACATGT ATAAGGTACG 360
   TATATAAAGT TATATGAATA ACTCAGTTAA TGATATAGAT GCTATAAGTG TATGTGTCTT 420
   AAAACTTCAT TTAGTTTACA TAATCTAAAA TACACTATTA AAAACACACC AGATTCTCAT 480
   ATATCCGGTG TGTTTGCAGT ATTATTCTTG ATAAACATCT ATTAAGGCGT TGGGATAATC 540
   ATCACGGTCT GCACTAAATA TATCTTTTGT TTTGAAATCA TCCACAATTG TACCATTATT 600
   TAACACAAAT CATACGANCA ACAATTTGAT TTAAAGTACT GCAAATATCA TGCGAGGGGG 660
   TATCATCGTA GTTGCTGGTC TTTA 684
(2) ANGABEN ZU SEQ ID NO: 10:
   (i) SEQUENZKENNZEICHEN:
      (A) LÄNGE: 915 Basenpaare
      (B) ART: Nucleotid
      (C) STRANGFORM: nicht bekannt
      (D) TOPOLOGIE: nicht bekannt
   (ii) ART DES MOLEKÜLS: cDNA
   (xi) SEQUENZBESCHREIBUNG: SEQ ID NO: 10:
   CCCTTATAGC AAAAGGAGCT TGAAACTTTG TCATGCTTTT ACTACTTGTT TCCATTGTTC    60
   ATATCACTCA AGTTTCCAAA TCCTCTGCAA ATACGCGTAA TCTTTTTATT GGATTTCGCA   120
   GCCATCGCAC CTATCAAAAT ATCAGCTGGA ATGACATTCA ACACTACTAC CAATCACTTG   180
   AATTAAAATA CTTCAAGCGT CTTTTATATA TCAGCATTTT GTTCGCATTG ATAGACATCA   240
   TTACGTTGCT GAAGTACCAT ACTGCTTGGT TAATCCTTGC AGAATTATGT ATTTACTTCA   300
   TTACACTTAT TTTGTTATCC AAAAAAGTAG AACATTATAT CGCACAAAAA GGAAAGTAGT   360
   TGTAATCCTA TCGAAAGAGG GAGAGTTCGT TCCAAAGTAG GATGGCAGCA GGTATTTAAT   420
   CCGCTATAAT TTAAGTTATT AAATTAGAAA GTCAGGTTTT TAATATGTCA GCAATCCACA   480
   GAAAATATAT TGCCTCTACT TTAATTATCT TAATAGCTTT AATGGTGCTC GTGAAATCGA   540
   ACCTGATTCT TTTCATAGAT GAGCCGGTAT ATCGCTTAGT GAGATTGCTT CGCATATCCC   600
   ATTTGCCAAT ACCTTCTTGT CCTATTACTC AGATATTTTT TCACCTTGGC ATATGGTTGG   660
   TGTAAAGGTT GTTATTTAAC GAATCTCTTA TTCAAAAATC GTCGTCTCGC TTATATTACA   720
   ACTATTTGGG CAACTTCCAC ATTATTGCTC GGCATTTGGT CTGAAATATT TTATCCACCG   780
   TCCAAGACCC AGTAGATTAT ATTTCAGGTT ATAGGTTCCC CAGCTTGGCA TACCATTGGG   840
   CCAATAGCCG GAGCGGCGGT GCCTTTTAAA TGCGGGTCCG GACGATTTTA TTGGCATAAT   900
   CCTGGTTATT AACGG                                                    915
(2) ANGABEN ZU SEQ ID NO: 11:
   (i) SEQUENZKENNZEICHEN:
      (A) LÄNGE: 5 Aminosäuren
      (B) ART: Aminosäure
      (C) STRANGFORM: nicht bekannt
      (D) TOPOLOGIE: nicht bekannt
   (ii) ART DES MOLEKÜLS: Peptid
   (iii) HYPOTHETISCH: NEIN
   (xi) SEQUENZBESCHREIBUNG: SEQ ID NO: 11:
   Leu Pro Xaa Thr Gly
   1 5
(2) ANGABEN ZU SEQ ID NO: 12:
   (i) SEQUENZKENNZEICHEN:
      (A) LÄNGE: 35 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 12:
      ATAAGGCGCC TTAGTTTAAT TATGCTTTGT GATTC 35
(2) ANGABEN ZU SEQ ID NO: 13:
   (i) SEQUENZKENNZEICHEN:
      (A) LÄNGE: 56 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 13:
      CGCAGGAAGC TTACCACAAT CTAAGAAATC TGAAATATCT CAAGCAAGTG GAGAAG 56
(2) ANGABEN ZU SEQ ID NO: 14:
   (i) SEQUENZKENNZEICHEN:
      (A) LÄNGE: 38 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 14:
      AATAAGGCGC CTCATTATCC ACCTGTTTCA GGTAGTTC 38
(2) ANGABEN ZU SEQ ID NO: 15:
   (i) SEQUENZKENNZEICHEN:
      (A) LÄNGE: 33 Basenpaare
      (B) ART: Nucleotid
      (C) STRANGFORM: nicht bekannt
      (D) TOPOLOGIE: linear
   (ii) ART DES MOLEKÜLS: Sonstige Nucleinsäure
   (iii) HYPOTHETISCH: NEIN
   (xi) SEQUENZBESCHREIBUNG: SEQ ID NO: 15:
      ACGAAAGCTT ACCACAATCT AAGAAATCTG AAC 33
(2) ANGABEN ZU SEQ ID NO: 16:
   (i) SEQUENZKENNZEICHEN:
      (A) LÄNGE: 3539 Basenpaare
      (B) ART: Nucleotid
      (C) STRANGFORM: nicht bekannt
      (D) TOPOLOGIE: nicht bekannt
   (ii) ART DES MOLEKÜLS: Sonstige Nucleinsäure
   (xi) SEQUENZBESCHREIBUNG: SEQ ID NO: 16:
   ATCACGTTAT CAAGTTTATC AATAGAATCA ACTTCCTGTG CTTTTTGGAA TAAAATCTTA 60
   ATTAATTCCA TTTGACGTTG TCCACGTTTT AAGTCTGAAT CATGATGTCT AGTTCTAGCA 120
   AACTGCTAAA GCCTCATCAC CATTTAATTT TTGGTACCCT ATTTTAATTT TAATCTTACC    180
   AGTATCATCT GTGTTAGGTT CATTTAAGTC GTATGGCACA TCATAGTATA TGCCACCAAG    240
   CTCATTTACA GCCTCGACAA ATGCTTTCAT ATTGACTCTC ACATAATAAT CAACAGGTAC    300
   ATTCATGGTA GCTTCTACCG AATCCATTGC GGCAATTGGA CCACCATATG CATGTGCATG    360
   GGTAATCTTA TCGTAATAGC CAACTTTAGG AATGTAGCTG ATAGTATCAC GTGGAATACT    420
   AAGCATTCTA ATTTGATGTT TTGATTGATT AAAAGTAGTT AAAATCATAG CGTCTGATCT    480
   AGAGTGTTCA GCATCCTGTC CTTTTTTTCT TCTTCCATCG TTATCATCGA TACCTAAGAA    540
   AAGAATAGAG ATAGGTTGTT CTTCGGGATT GACTTTATTA TCTCTTAAGT TGGATTGACG    600
   ATTAGCATTT TTGCTGTCTA GAGAAGATTC GAATGCATCT TGGGACGTTT TAAAAAGTAA    660
   CGTAGCGAAG ACTATTGGAA CAACAATGAG AACCAATGCT AGAAGGATCA AAAAGTATTT    720
   TAAAAATTTA TTCATGATTG ATGCTCCTAT ATTAAATTTT TGTTAAATCA TAATCATAAT    780
   TTTAAAATAT AATTAGTTGA ACGATATTTA TACAACTTCA TTACTAATAC ATTATAGCCT    840
   TAAATTGTAG TACTTTATAG TAAAAAATAC AAATGTTATG TCAAAGTTCC TTGTAATTTG    900
   AATGTTTTGT TAAAATCAAA AAGCATTTTT AAAAATATAA TGCGACTTTA GTATGATGTC    960
   TTAAAAAATT TAAAGGCGTA ATATAAAATC GAGTGATTTA TACAAAGTGA TAAGGTATAA   1020
   TTAATTTTGT TGTATAGATA ACATATTAGA AAAACTTATT ACTATGTAGC GTAAAGCATA   1080
   AATGAATGAT AAGTTAATTT TTGTAAGTTG TATTATTAGG CATCTAGGTT CAAATTTTTA   1140
   ACGATCTAAA GACATAAAAT GAATACATTG TGAGGCAATT ATCAAATGAA ATTTAATAAA   1200
   NNNNTTTTTG TGTACTATTA TTTATCATTA TTTCTATAGC GTTAATTTTT CATCGATTAC   1260
   AGACGAAGAC ACATTCTATA GACCCAATAC ATAAGGAAAC AAAATTATCA GACAATGAAA   1320
   AATATTTAGT GGATCGTAAT AAGGAAAAGG TTGCGCCGTC TAAACTAAAA GAGGTATATA   1380
   ATAGCAAGGA TCCTAAATAT AAGAAAATTG ACAAGTATTT ACAAAGTTCA TTATTTAACG   1440
   GTTCAGTAGC TATATATGAA AATGGCAAAT TGAAAATGAG TAAAGGTTAT GGATATCAAG   1500
   ATTTTGAAAA AGGTATTAAA AACACACCGA ATACGATGTT TTTAATAGGT TCAGCTCAAA 1560
   AATTTTCAAC AGGGTTACTG TTAAAACAGT TAGAAGAAGA ACATAAAATA AATATCAATG 1620
   ATCCAGTAAG TAAATACCTT CCATGGTTTA AAACATCTAA GCCTATCCCA TTGAAAGATT 1680
   TAATGTTGCA TCAAAGTGGA TTATATAAAT ATAAATCCTC AAAAGATTAT AAAAATTTAG 1740
   ATCAAGCAGT TAAAGCGATT CAAAAACGTG GTATTGATCC TAAGAAATAC AAAAAGCATA 1800
   TGTATAACGA TGGGAATTAT TTAGTACTTG CGAAAGTAAT TGAAGAAGTT ACAGGTAAAT 1860
   CTTATGCTGA GAATTATTAT ACAAAAATAG GAGATCCTTT AAAACTTCAG CACACAGCAT 1920
   TTTATGATGA ACAACCTTTT AAAAAATATC TAGCAAAAGG TTATGCTTAT AATAGTACAG 1980
   GACTTTCATT CTTAAGACCT AATATTTTGG ACCAATACTA TGGTGCAGGT AATTTATATA 2040
   TGACACCAAC AGATATGGGT AAATTAATTA CTCAAATACA ACAATATAAA TTATTCAGTC 2100
   CTAAAATAAC CAATCCATTA TTACATGAGT TTGGTACGAA ACAGTATCCA GATGAATATC 2160
   GATATGGTTT CTATGCTAAG CCAACATTGA ATAGACTTAA CGGGGGATTC TTTGGACAAG 2220
   TCTTTACTGT TTACTATAAT GATAAGTATG TAGTTGTACT TGCATTAAAT GTAAAAGGAA 2280
   ACAATGAAGT TCGAATCAAA CATATTTATA ATGATATTTT AAAACAAAAT AAACCTTACA 2340
   ATACGAAGGG TGTTATTGTT CAATAATTAA TATAGAAGAT ATAACATGTA TATGGCATTA 2400
   AGGCATCGAC CTTATCTGAC CAGTATACGA GTTATATCTT CTTTTTTATA GTGGTAAAAA 2460
   GTTTAAAGTA TAAGGTTGAA GAAGGATGAG TTTAAAAATA TGTGTTAACT GATAAAAGGG 2520
   GAAATCATTT GGTGAGTTGG CATCAGACTA AAATGAATGA AGACGAATAC CTTGGTCCAT 2580
   GCGTGGTGTG AATGTTTTCT AAATCTAACT GGAAATGATA ATTTAATAAT GTACACTTTT 2640
   GATTGTTTAA ACATGTACAA TTTTTAAAAA CCTAGGGATA ATTTTATTGA CGTGAACAAC 2700
   ATTGAGATTA TCTTTAGACA TAAATATGGA TTTTGAAGTT ACTATATTTA TTAGTTGTGG 2760
   GTGTTAATGT ATTAATTGAA TTGATTATAA TGATTGTCTT ATAAGATATA GAAGGGGACT 2820
   TAACCATTTA AAAATTGAAG GGAAATAAAA ACCAGCTAAC AAAAGTGTTA GCTGGTTAAA 2880
   CTTTACATTA TTAAAATGTT GTTTATAAGT GTGAAGAGTG ACCGCCTTGC ATAACCCAAT 2940
   ATGTTCCGAC AACGAAACAA AGTGTAATTA CAAGAGCAAA GATAACTTTG AATGTTTGTA 3000
   AAGCGTCCAT CTTTACCTTC AGTTAAATGC ATGAACATTA ATAATTGAAG TCGCTGCTTG 3060
   GACGAATGCA AAGCCAAAGA TAATTGTCAA CTTCGCGTGG AATGTTAATG ACGGTGTATA 3120
   GTGTTACGTA AACTGCTAAA AGCGTTAATA CGATAGATGC GATAAATCCT ACAGTATGTT 3180
   TCATTATTGT ACTCATCCGC TATACATCCA TCCCTATCAT ATATACGGCA GTAAAGATGA 3240
   AAACCCAAAG CGAACATCTA AGAAGTGCCA GTATAAACTT ACTATAAATA ATTTTGGCGC 3300
   ATTATATTTG TCTAATCCGC GTCGTTGGAT TTGGATGTAA TAAACAATAT GGCCCAAGAC 3360
   GATACCTAGC GATACGTGAC AACCATGCGT TCCTAATAGG ATAAAGAAAC TAGACCGAGT 3420
   AAGAACCAAT TGTTGGGTTA ACGCGCTTCT GATGCGATAG TGTGCGAATT CGATAAATTT 3480
   CGAATGCCAA CAAAGACTAA ACCTAAAAGT AACGTAATGA TCATCCAAAA CATCATTAA 3539
(2) ANGABEN ZU SEQ ID NO: 17:
   (i) SEQUENZKENNZEICHEN:
      (A) LÄNGE: 1474 Basenpaare
      (B) ART: Nucleotid
      (C) STRANGFORM: nicht bekannt
      (D) TOPOLOGIE: nicht bekannt
   (ii) ART DES MOLEKÜLS: Sonstige Nucleinsäure
   (xi) SEQUENZBESCHREIBUNG: SEQ ID NO: 17:
   TAGATGAAAG TGAGGACCGC GTATGACGGA AAACAAAGGA TCTTCTCAGC CTAAGAAAAA 60
   CGGTAATAAT GGTGCCAAAT CCAACTCAAA AAAGAATAGA AATGTGAAGA GAACGATTAT 120
   TAAGATTATT GGCTTCATGA TTATTGCATT TTTTGTTGTT CTTTTACTAG GTATCTTATT 180
   GTTTGCTTAT TATGCTTGGA AAGCACCTGC ATTTACCGAA GCTAAATTAC AAGATCCGAT 240
   NNNNNNAAAG ATATATGACA AGAACGGAGA ACTTGTAAAA ACATTAGATA ATGGCCAAAG 300
   ACATGAGCAT GTAAATTTAA AAGACGTGNN GAAATCAATG AAAGACGCAG TACTTGCAAC 360
   TGAAGACAAT CGTTTCTACG AACATGGCGC ACTTGATTAT AAACGTTTAT TCGGTGCAAT   420
   TGGTAAGAAC TTGACTGGTG GATTTGGTTC TGAAGGTGCC TCAACATTAA CACAACAAGT   480
   TGTTAAAGAT GCATTTTTAN CAAAACATAA ATCTATTGGA CGTAAAGCTC AAGAAGCATA   540
   CTTATCATAT CGTTTAGAAC AAGAGTATAG TAAAGATGAT ATCTTCCAAG TATATCTAAA   600
   CAAAATTTAC TATTCTGATG GCGTAANNNN NNNNNNNNNN NNNNNNNNNN NNNNNNNNNN   660
   TAACGATGCA CCTAAGAAAT TCGCTGCCAA ACTTGGCTTA AACTACGAAG GCGATATTGG   720
   TCCATCTGAA GTACTTGGTG GTTCTGCTTC AGAATTCTCA CAAACACAAT TAGCATCAGC   780
   ATTTGCTGCA ATCGCTAACG GTGGTACTTA TAACAACGCG CATTCAATTC AAAAAGTAGT   840
   TACTCGTGAC NNTGAAACAA TCGAATACGA TCATACTAGC CATAAAGCGA TGAGTGATTA   900
   CACTGCATAC ATGTTAGCTG AGATGCTAAA AGGTACATTT AAACCATATG GTTCTGCATA   960
   TGGCCATGGT GTAGCTGGAG TAAATATTGG TGCTAAGACA GGTACTGGTA CTTACGGTGC  1020
   TGAAACTTAT TCACAATATA ATTTACCTGA TAATGCAGCG AAAGCAGTGT GGATTAACGG  1080
   CTTTACACCT CAATACACTA TGTCAGTGTG GATGGGCTTC AGTAAAGTTA AACAATATGG  1140
   TGAAAACTCA TTTGTGGGAC ATAGCCAACA AGAATATCCA CAGTTCTTAT ATGAAAATGT  1200
   GATGTCAAAA ATTTCATCTA GACATGGCGA AGACTTTAAA CGTCCTAGCT CANTAAGTGG  1260
   TAGTATCCCA TNCAATCAAT GTTTCTGGTA GTCAAGATAA CAACACTACA AATCGTAGTA  1320
   CACACGGTGG TAGTGACACA TCAGAAACAG CAGTGGTACT GCACAATCAA ATAACAATAC  1380
   TAGATCTCAA CAATCTAGAA ACAGCGGTGG ATTAACAGGT ATATTCAACT AATCCACACA  1440
   ACATAAAATC CTCAGTTATA CCATATTTAT GGCG                              1474

## Beschreibung

Gegenstand der vorliegenden Erfindung ist ein Verfahren zum Identifizieren einer Nukleinsäure, die für einen Polypeptidfaktor kodiert, welcher die kovalente Verknüpfung von Polypeptiden an die Oberfläche von Gram-positiven Bakterien beeinflußt sowie die nach diesem Verfahren erhältlichen Nukleinsäuren.

Angesichts des vermehrten Auftretens antibiotikaresistenter Stämme stellen durch Gram-positive Bakterien hervorgerufene Infektionen beim Menschen eine zunehmende therapeutische Herausforderung dar. Eine Vielzahl von bakteriellen Oberflächenproteinen ist mit der Pathogenese dieser Organismen verbunden. So sind zellwandverankerte Pathogenitätsfaktoren bekannt, welche die bakterielle Adhäsion durch Bindung an extrazelluläre Matrixkomponenten der Wirtsgewebe, wie z.B. Kollagen, fördern. Andere Faktoren binden Serumkomponenten wie IgG und verbergen somit die authentische Bakterienoberfläche vor dem Immunsystem des Wirtes. Eine gezielte Hemmung der Verknüpfungsreaktion dieser Proteine an die bakterielle Zellwand ist daher von großem medizinischen Interesse.

Schneewind et al. (Cell, Vol. 70, p. 267 - 281, 1992) untersuchen den Verankerungsmechanismus von Protein A in der Zellwand von Staphylokokken. Protein A gehört zu einer wachsenden Klasse von Oberflächenproteinen Gram-positiver Bakterien, welche durch eine Abfolge des charakteristischen Sequenzmotives LPXTG, gefolgt von einer Gruppe aus 15 - 22 hydrophoben Aminosäuren sowie einer am C-terminalen Ende befindlichen Gruppe aus 5 - 12 geladenen Aminosäuren gekennzeichnet sind. Die Konservierung dieser Elemente wird als Indikation eines gemeinsamen Ausschleusungsmechanismus dieser Proteine in unterschiedlichen Gram-positiven Spezies angesehen. Um die Lokalisation des Protein A (Unterscheidung zwischen in der Zellwand verankertem und sezerniertem Protein A) in *S*. *aureus* feststellen zu können, greifen die Autoren auf radioaktive Labellingverfahren zurück. Die Bedeutung der oben genannten Sequenzelemente für die Zellwandverankerung wird mit Hilfe von Hypridproteinen sowie durch Mutagenese des LPXTG-Motives und des C-Terminus untermauert. Schneewind et al. befassen sich jedoch nicht mit möglicherweise die Verankerung der Oberflächenproteine katalysierenden Enzymen noch mit deren Hemmung.

Die Zellwandverankerungselemente in Oberflächenproteinen Gram-positiver Bakterien sind ebenfalls Thema eines weiteren Artikels von Schneewind et al. (EMBO J., Vol. 12, p. 4803 - 4811, 1993). Es wird gezeigt, daß Enterotoxin B, ein normalerweise in das Medium sezerniertes Protein, mittels C-terminaler Fusion an das Protein A-Verankerungssignal in der Staphylokokken-Zellwand verankert werden kann. Die Ergebnisse unterstützen die Hypothese, daß das Zellwand-Sorting mit einer proteolytischen Spaltung der Polypeptidkette am C-Terminus einhergeht. Vermutlich ist das LPXTG-Motiv die Stelle dieser Spaltung und kovalenter Zellwandverknüpfung, während der geladene Sequenzabschnitt als Retentionssignal während des Zellwand-Sortings dient. Die Relevanz der faltungsbedingten geometrischen Länge der hydrophoben Domäne wird experimentell bestätigt.

Mit dem Zelloberflächendisplay rekombinanter Proteine auf *Staphylococcus carnosus* befaßt sich ein Artikel von Samuelson et al. (J. Bacteriol., Vol. 177, no. 6, p. 1470 - 1476, 1995). Der Oberflächendisplay des Malariapeptides M3 erfolgt hier unter Verwendung von Promoter, Sekretionssignal und Propeptidregion des Lipasegens von *S*. *hyicus* sowie der Zellwandverankerungsregionen des Protein A von *S. aureus.* Die hybride Proteinstruktur beinhaltet ferner ein Serumalbuminbindeprotein, welches der Detektion der rekombinanten, oberflächen-verankerten Proteine in einem colorimetrischen Sandwichassay dient. Weitere Detektionsmethoden umfassen die Immunogold-Elektronenmikroskopie, Immunofluoreszenzassays sowie das fluoreszenzaktivierte Cellsorting (FACS). Auch Samuelson et al. befassen sich nicht mit den genauen molekularen Mechanismen der Zellwandverankerung.

Die Struktur des Zellwandankers der Oberflächenproteine in *Staphylococcus aureus* ist Thema eines Reports von Schneewind et al. (Science, Vol. 268, p. 103 - 106, 1995). Die Autoren verwenden eine Kombination molekularbiologischer und massenspektrometrischer Techniken und können zeigen, daß nach Spaltung des Oberflächenproteins zwischen Threonin und Glycin des konservierten LPXTG-Motives die Carboxylgruppe des Threonins durch Transpeptidierung mit der freien Aminogruppe des Zellwand-Pentaglycins kovalent an den Mureinsacculus gebunden wird. Das für die Proteolyse und Transpeptidierung vermutlich verantwortliche Protein, die sogenannte Sortase, wird jedoch auch von Schneewind et al. nicht identifiziert bzw. charakterisiert.

Strauß und Götz (Molecular Microbiology, Vol. 21, p. 491 - 500, 1996) befassen sich mit der in vivo Immobilisierung enzymatisch aktiver Polypeptide auf der Zelloberfläche von *Staphylococcus carnosus.* Sie konstruieren ein Hybridprotein, welches aus *Staphylococcus hyicus* Lipase und der C-terminalen Region des *Staphylococcus aureus* Fibronectin Bindeprotein B (FnBPB) besteht. Um die Zellwandassoziation der Prolipase bzw. des proLipFnBPB-Hybrides zu untersuchen, verwenden die Autoren ein prolipasespezifisches Antiserum in einem Immunofluoreszenzassay sowie Immunoblotting. Weitere Untersuchungen belegen, daß für eine effiziente Faltung der Lipase in ihre aktive Konformation offenbar ein Abstand von ca. 90 Aminosäuren zwischen dem C-Terminus des Enzyms und dem Zellwand-Sortingsignal unerläßlich ist. Der Einfluß größerer Abstände wurde an Fusionen aus proLip und der C-terminalen Region von *S. aureus* Protein A (proLipSPA, Spacer mit 165 Aminosäuren) und *S*. *aureus* Fibronectin Bindeprotein A (proLipFnBPA, Spacer mit 223 Aminosäuren) untersucht. Zusätzliche Experimente wurden mit *E. coli* β-Lactamase als Reportermolekül durchgeführt.

Die internationale Patentanmeldung PCT/US 96/14154 (Internationale Veröffentlichungsnummer WO 97/08553) beschreibt ein Verfahren zum stabilen, nicht-kovalenten Display von Proteinen, Peptiden und anderen Substanzen auf der Oberfläche von Gram-positiven Bakterien. Es wurden vergleichende Untersuchungen zwischen dem nicht-kovalenten sowie den oben näher beschriebenen kovalenten Displayverfahren durchgeführt. Bei Ersatz des C-terminalen Sorting-Signals von Protein A, welches ein kovalentes Display generiert, durch das Zellwand Targeting-Signal von Lysostaphin (SPA_{CWT}) konnte eine im wesentlichen unveränderte Bindungsintensität von FITC-markiertem IgG an die Staphylokokken-Oberfläche beobachtet werden.

Das US-Patent 5,616,686 offenbart ein aus ca. 6 bis 20 Aminosäuren bestehendes Polypeptid, welches als integralen Bestandteil ein für die Verankerung von virulenzdeterminierenden Proteinen auf der Oberfläche von Gram-positiven Bakterien verantwortliches Peptidkonstrukt enthält. Insbesondere ist dieses Konstrukt dadurch gekennzeichnet, daß es an den Positionen 1, 2, 4 und 5 der Aminosäuresequenz die Aminosäuren L, P, T und G aufweist. Aufgrund der Homologie dieser Peptide mit den Sequenzen der Virulenzdeterminanten in den Wildtyp-Oberflächenproteinen reagieren erstere vermutlich mit den an der Verankerung beteiligten Enzymen. Hieraus resultiert, daß die Virulenzdeterminanten der Bakterien nicht oder nur in geringerem Maße verankert werden können und so ein Fortschreiten der Infektion unterbunden wird. Das bzw. die an der Oberflächenverankerung beteiligte(n) Enzym bzw. Enzyme werden jedoch auch in dieser Patentschrift nicht charakterisiert.

Die internationale Patentanmeldung PCT/US 93/02355 (internationale Veröffentlichungsnummer WO 93/18163) befaßt sich mit der Bereitstellung von Fusionsproteinen, welche mindestens die Ankerregion Gram-positiver Oberflächenproteine sowie variable Proteine, Polypeptide oder Peptide mit insbesondere therapeutischer Wirkung in Mensch und Tier enthalten. Die Ankerregion umfaßt ein LPSTGE-Segment, ein Spacer-Segment der Sequenz TAN, ein aus 20 Aminosäuren bestehendes hydrophobes Segment sowie einen geladenen Segmentabschnitt mit der Sequenz KRKEEN.

Es ist daher von Interesse, ein Verfahren bereitzustellen, welches die Identifizierung von Nukleinsäuren in Gram-positiven Bakterien erlaubt, die für solche Polypeptidfaktoren kodieren, die direkt oder indirekt die kovalente Verknüpfung von Polypeptiden an die Oberfläche der Gram-positiven Bakterien beeinflussen.

Überraschenderweise wird dieses Ziel durch das Verfahren der vorliegenden Erfindung erreicht.

Polypeptide im Sinne der Erfindung bezeichnet aus in der Regel mindestens 20 Aminosäuren aufgebaute Polymere und umfaßt insbesondere auch Proteine. Die Aminosäuren sind durch den 1-Buchstaben-Code dargestellt, wobei X für eine beliebige Aminosäure steht.

Zunächst soll im folgenden die Grundlage einer bevorzugten Ausführungsform des erfindungsgemäßen Verfahrens dargestellt werden, bevor im einzelnen auf das Verfahren zur Identifizierung von Nukleinsäuren eingegangen wird.

Das erfindungsgemäße Verfahren ist in einer bevorzugten Ausführungsform als enzymatischer Reporterassay aufzufassen, der die Auswirkung von Mutationen auf bakterielle Faktoren (Targets) erfaßt, welche direkt oder indirekt an der von einem LPXTG-Motiv abhängigen C-terminalen Verankerung von Polypeptiden an der Oberfläche von Gram-positiven Bakterien mitwirken. Aus der Vielzahl von Faktoren und Vorgängen, die sich auf diesen Prozeß auswirken können, zielt das vorliegende Verfahren vorzugsweise auf solche enzymatischen Schritte, die nach dem Beginn der Translokation der Zelloberflächen-Polypeptide über die Cytoplasmamembran stattfinden. Darüber hinaus erfaßt das erfindungsgemäße Verfahren zum Teil enzymatische und andere Targets, die an der Biosynthese des Zellwandmureins mitwirken. Anhand der phänotypischen Merkmale der im jeweiligen Verfahren verwendeten Zellen lassen sich Mutationen bestimmten Targetgruppen zuordnen.

In einer besonders bevorzugten Ausführungsform des erfindungsgemäßen Verfahrens bildet die zelluläre und molekulare Grundlage des Reporterassays ein rekombinanter *Staphylococcus carnosus* Klon, welcher ein selektierbares Expressionsplasmid mit einer induzierbaren Reportergenfusion enthält. Die Genfusion kodiert für ein Hybridpolypeptid bestehend aus einem N-terminalen Signalpeptid, einem Vorläuferprotein der Lipase aus *Staphylococcus hyicus* und einem C-terminalen Anteil des Fibronektin-Bindeproteins B (FnBPB) aus *Staphylococcus aureus.* Nach seiner Produktion im Cytoplasma wird das Hybridpolypeptid aufgrund seiner N-terminalen Signalstrukcuren durch die bakterielle Zellmembran transportiert und am Aminoende durch eine Signalpeptidase prozessiert. Ferner erfolgt eine Spaltung im C-terminalen LPXTG-Erkennungsmotiv und das verbleibende Hybridprotein wird kovalent mit dem Murein verknüpft. Auf experimentellem Wege wurde ermittelt, daß unterschiedliche Längen des FnBPB-Anteils die Ausbildung enzymatischer Aktivität der Lipasefusionen in unterschiedlicher Weise beeinflussen. Ein Konstrukt wurde identifiziert, das in seiner Zelloberflächen-gebundenen Form keine Lipaseaktivität aufwies (kodiert von Plasmid pTX30Δ82). Wurde die entsprechende Fusion jedoch nach ihrer kovalenten Verankerung durch Lysostaphinbehandlung von der Bakterienoberfläche freigesetzt, wurde die volle Lipaseaktivität erzielt. Im Rahmen der vorliegenden Erfindung wurde erstmals erkannt, daß in dem betreffenden Assayklon Störungen in der Zellwandverankerung der Lipasefusion zur Freisetzung der Fusion verbunden mit dem Auftreten von Lipaseaktivität in den Kulturüberständen führen. Als mögliche Angriffspunkte kommen verschiedene zelluläre Faktoren in Betracht, die entsprechend ihrem Wachstumsverhalten im wesentlichen in zwei Gruppen eingeteilt werden können.

Ein Target bzw. eine Targetgruppe ist das als Sortase bezeichnete Enzym bzw. der Enzymkomplex, das bzw. der an der Oberfläche der Bakterien die carboxyterminale Spaltung relevanter Polypeptide im LPXTG-Motiv und deren anschließende kovalente Verknüpfung an Peptidbestandteile des Zellwandmureins wie z.B. Interpeptidbrücken, insbesondere Pentaglycineinheiten, bewerkstelligt. Die Inhibierung dieser Funktionen führt zwar vermutlich zur Freisetzung oberflächengebundener Faktoren und damit vermutlich zur Attenuierung pathogener Bakterien, wohl aber nicht zu maßgeblichen Beeinträchtigungen in der Lebens- und Teilungsfähigkeit der Bakterien. Kennzeichnendes phänotypisches Merkmal dieser Targetgruppe im Assayverfahren ist die Freisetzung von Lipaseaktivität in das Assaymedium bei mehr oder weniger unbeeinträchtigtem Wachstumsverhalten der Bakterien. Im Gegensatz dazu führt die Beeinträchtigung eines Targets bzw. einer Targetgruppe mit essentieller Funktion in der Mureinsynthese zu massiven phänotypisch erfaßbaren Veränderungen in der Lebens- und Teilungsfähigkeit der Bakterien.

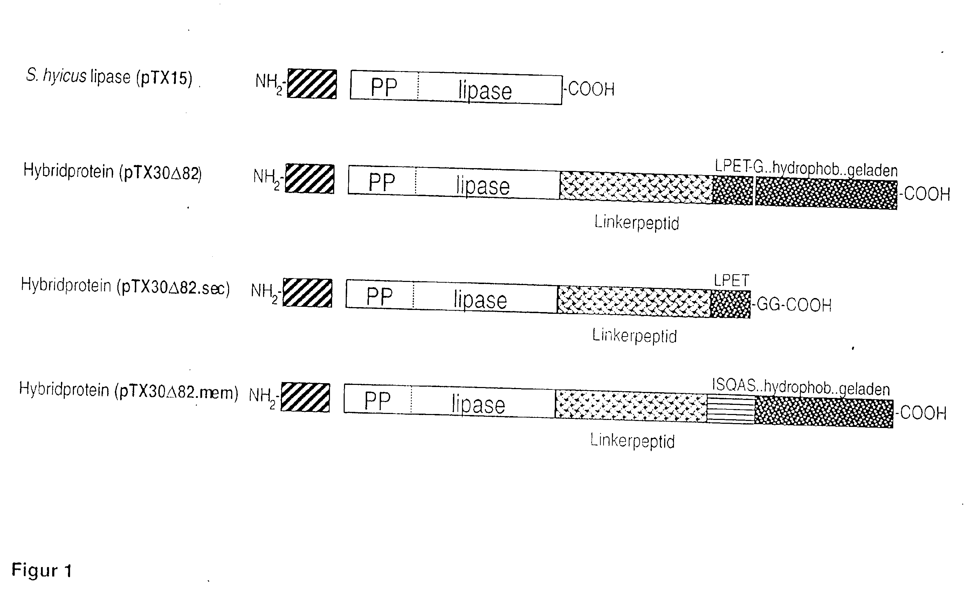

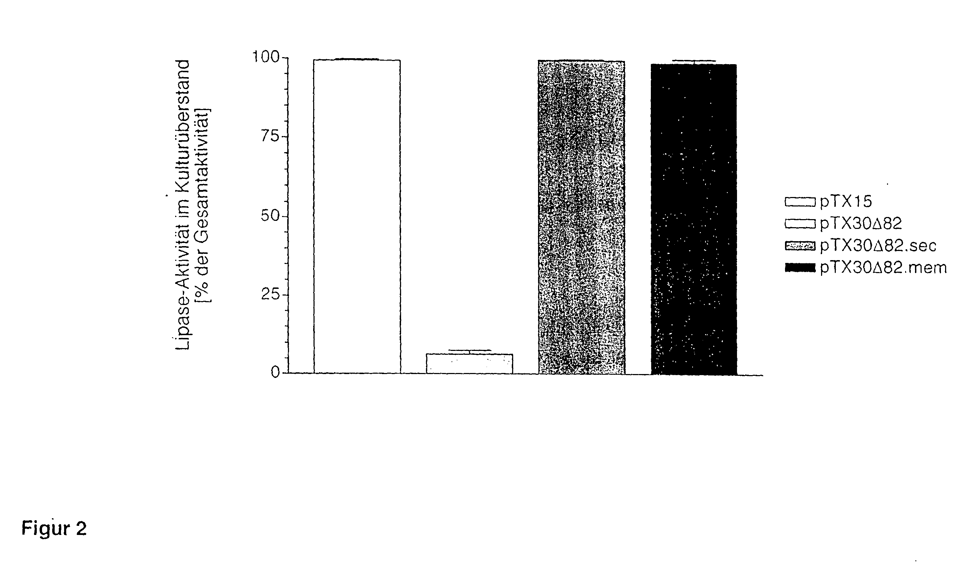

Um die potentielle Eignung des erfindungsgemäßen Verfahrens für die Untersuchung des Targets "Sortase" zu demonstrieren, wurden zwei unterschiedliche Hemm-/Mutations-Szenarien mit Hilfe gentechnisch gezielt veränderter Hybridproteine simuliert (siehe hierzu Ausführungsbeispiel 1 sowie Figuren 1 und 2).

*S. carnosus*/ pTX30Δ82.mem produziert nach Induktion des Xylose-Promoters ein Hybridprotein bestehend aus der *S. hyicus* Prolipase und dem C-terminalen Fragment des *S. aureus* Fibronectin-Bindeprotein B (FnBPB), wobei das für die Verankerung mit der Zellwand wichtige LPXTG-Motiv gegen die Sequenz ISQAS ausgetauscht wurde (Figur 1). In anderen Ausführungsformen kann jedoch auch ein Austausch gegen eine beliebige andere, bevorzugt aus 5 Aminosäuren bestehende Sequenz erfolgen. In einer weiteren Ausführungsform ist für den mem-Phänotyp eine LPXTG-Substitution nicht notwendig, sondern auch eine komplette Deletion des LPXTG-Motivs denkbar. Damit entfällt die spezifische Spaltung am LPXTG-Motiv und somit die kovalente Verknüpfung an den Mureinsacculus. Überraschenderweise konnte gezeigt werden, daß die hydrophobe Ankersequenz, die somit am Carboxylende der Fusion erhalten bleibt, offenbar nicht ausreichend ist, um die Lipase stabil und insbesondere in inaktiver Form in der Zellhülle und somit an der Zelloberfläche zu verankern. Es kommt zur quantitativen Freisetzung der Lipaseaktivität von der Bakterienoberfläche in das Kulturmedium. Dieser Klon simuliert somit die Inhibition der Spaltungsreaktion durch die Sortase, die als wesentlicher Schritt der kovalenten Verknüpfung des N-terminalen Spaltprodukts an das Murein der Zellwand vorangeht.

*S. carnosus*/ pTX30Δ82.sec produziert nach Induktion des Xylose-Promoters ein Hybridprotein bestehend aus der *S. hyicus* Prolipase und einem C-terminalen Fragment des *S. aureus* Fibronectin-Bindeprotein B (FnBPB), welches mit dem Motiv LPETGG endet (Figur 1). Dieser Klon simuliert die Inhibition der (kovalenten) Verknüpfungsreaktion zwischen dem zu verankernden, C-terminal bereits prozessierten Lipase-Hybridprotein und der Zellwand. Das Lipase-Hybridprotein wird von diesem Klon quantitativ in aktiver Konformation in den Kulturüberstand freigesetzt.

Die Erfindung betrifft ein Verfahren zum Identifizieren einer Nukleinsäure, die für einen Polypeptidfaktor kodiert, welcher die kovalente Verknüpfung von Polypeptiden an die Oberfläche von Gram-positiven Bakterien beeinflußt, mit folgenden Schritten:
a) Bereitstellen einer Probe Gram-positiver Bakterien, welche genetisch veränderbar sind und mindestens eine kovalent mit der Oberfläche der Gram-positiven Bakterien verbundene oder verbindbare enzymatische Reportersubstanz aufweisen oder bilden, wobei die mindestens eine Reportersubstanz ohne kovalente Bindung an die Oberfläche der Gram-positiven Bakterien eine andere enzymatische Aktivität aufweist als bei kovalenter Bindung an die Oberfläche der Gram-positiven Bakterien,
b) Verursachen von genetischen Veränderungen in Gram-positiven Bakterien der Probe,
c) Bestimmen der enzymatischen Aktivität der Reportersubstanz der Gram-positiven Bakterien der Probe,
d) Separieren von Gram-positiven Bakterien, die eine andere enzymatische Aktivität der Reportersubstanz aufweisen als die, die bei kovalenter Bindung der Reportersubstanz an die Oberfläche der Gram-positiven Bakterien auftritt,
e) Isolieren der Nukleinsäure der in Schritt d) separierten Gram-positiven Bakterien,
f) Identifizieren mindestens eines Abschnittes der in Schritt e) isolierten Nukleinsäure, der die genetische Veränderung trägt,
g) Identifizieren einer Nukleinsäure, die für einen Polypeptidfaktor kodiert, welcher die kovalente Verknüpfung von Polypeptiden an die Oberfläche von Gram-positiven Bakterien beeinflußt, auf der Grundlage des in Schritt f) identifizierten Abschnittes.

Im Sinne des erfindungsgemäßen Verfahrens kann "ohne kovalente Bindung" sowohl nicht-kovalente Bindung an die Oberfläche der Gram-positiven Bakterien als auch vollständige Freisetzung bedeuten.

Die Bestimmung der enzymatischen Aktivität der mindestens einen Reportersubstanz der Gram-positiven Bakterien der Probe kann in bevorzugter Weise im Vergleich zu mindestens einer Referenzprobe, die nicht genetisch verändert wurde, und/oder mindestens einer Referenzprobe, in der die Reportersubstanz nicht-kovalent an die Oberfläche Gram-positiver Bakterien gebunden ist, und/oder mindestens einer Referenzprobe, in der die Reportersubstanz kovalent an die Oberfläche der Gram-positiven Bakterien gebunden ist, und/oder mindestens einer Referenzprobe, in der die Reportersubstanz ohne kovalente Bindung an die Oberfläche der Gram-positiven Bakterien vorliegt, erfolgen.

Es kann bevorzugt sein, die gemäß Schritt g) des Verfahrens identifizierte Nukleinsäure zu gewinnen. Die von dieser Nukleinsäure kodierten Polypeptide können insbesondere im Wirkstoffscreening Verwendung finden.

Die kovalente Verknüpfung der Polypeptide erfolgt, wie bereits beschrieben, bevorzugt an das Murein der Zellwand, insbesondere an Interpeptidbrücken wie z.B. Pentaglycine der Zellwand Gram-positiver Bakterien. Bei den Polypeptiden handelt es sich insbesondere um Pathogenitätsfaktoren der Gram-positiven Bakterien.

Das erfindungsgemäße Verfahren erlaubt in vorteilhafter Weise selektiv die Identifizierung einer Nukleinsäure, die für einen Polypeptidfaktor kodiert, welcher die kovalente Verknüpfung von Polypeptiden an die Oberfläche von Gram-positiven Bakterien direkt oder indirekt beeinflußt. Wie eingangs erläutert, umfaßt der putative Prozeß der Oberflächenverankerung zwei spezifische Schritte:
- a): Spaltung zwischen Threonin und Glycin des LPXTG-Motives und
- b): kovalente Verknüpfung des Threonins an Peptidbestandteile, insbesondere an die Interpeptidbrücke, der Zellwand.

Mittels des erfindungsgemäßen Verfahrens werden somit insbesondere solche Nukleinsäuren erfaßt, die für Polypeptidfaktoren kodieren, die
- die Spaltungsreaktion ausführen,
- eine kovalente Verknüpfung der Polypeptide durchführen.
- die für die Integrität und dem korrekten Aufbau der Zellwand wichtig sind.

In einer bevorzugten Ausführungsform des erfindungsgemäßen Verfahrens wird als Reportersubstanz ein Hybridpolypeptid verwendet, welches insbesondere die folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt. Wie in den nachfolgenden Beispielen gezeigt, kann insbesondere als enzymatischer Bestandteil des Hybridpolypeptides *S. hyicus* Lipase verwendet werden. Es kann jedoch auch von Vorteil sein, *E. coli* β-Lactamase oder andere Enzyme zu verwenden. Natürlich vorkommende Oberflächenpolypeptide können ebenso wie gentechnische hergestellte Hybridpolypeptide als Reportersubstanzen dienen, die infolge der Wirkung geeigneter genetischer Veränderungen in den zu untersuchenden Bakterien im Medium mit Hilfe der gesamten Palette der bekannten chemischen, biochemischen und immunologischen Methoden nachgewiesen werden können. Um allerdings einen hohen Probendurchsatz bei einer ungeminderten Verläßlichkeit des erfindungsgemäßen Verfahrens zu gewährleisten, ist es insbesondere vorteilhaft, als Reportersubstanzen Hybridpolypeptide mit enzymatischer Aktivität zu verwenden.

Es kann drüber hinaus bevorzugt sein, Reportersubstanzen mit mindestens einer detektierbaren Eigenschaft zu verwenden, wobei die Reportersubstanz ohne kovalente Bindung an die Oberfläche der Gram-positiven Bakterien eine veränderte detektierbare Eigenschaft aufweist als bei kovalenter Bindung an die Oberfläche der Gram-positiven Bakterien.

Es kann weiterhin bevorzugt sein, das Enzym als Proenzym bereitzustellen.

Darüber hinaus kann es insbesondere bevorzugt sein, die Änderung der enzymatischen Aktivität durch den Übergang des Enzyms aus einer inaktiven in eine aktive Konformation oder umgekehrt, zu bestimmen. Dies kann bevorzugt durch Verwendung eines zwischen Enzym und LPXTG-Motiv angeordneten Linkerpeptides erzielt werden.

In einer bevorzugten Ausführungsform des erfindungsgemäßen Verfahrens ist die Anzahl der Aminosäuren des Linkerpeptides so gewählt, daß das Enzym in einer inaktiven Konformation an der Oberfläche der Gram-positiven Bakterien verankert wird. Bei Verwendung von *S*. *hyicus* Lipase sollte die Anzahl der Aminosäuren im Linkerpeptid insbesondere weniger als zehn betragen. In einer weiteren Ausführungsform könnte unter Verzicht auf das Linkerpeptid das Enzym mit seinem C-Terminus direkt an das LPXTG-Motiv fusioniert vorliegen. Die Gram-positiven Bakterien tragen somit kovalent an ihrer Oberfläche gebundene inaktive Enzyme, welche bei nicht-kovalenter Verknüpfung, insbesondere bei Freisetzung von der Oberfläche der Gram-positiven Bakterien, in eine aktive Konformation falten und somit eine bestimmbare Veränderung hinsichtlich ihrer enzymatischen Aktivität, erfahren.

Es ist insbesondere bevorzugt, das erfindungsgemäße Verfahren mit solchen Gram-positiven Bakterien durchzuführen, die einen geringen natürlichen Zellwand-turn-over und/oder eine geringe Anzahl an Zellwandproteasen und/oder eine geringe Anzahl an sekretierten Proteasen aufweisen, um den Anteil an falschpositiven Ergebnissen zu minimieren. Somit kann das erfindungsgemäße Verfahren nicht nur mit natürlich vorkommenden Gram-positiven Bakterien wie z.B. *S*. *carnosus* durchgeführt werden, sondern auch mit bereits genetisch veränderten Bakterien.

Darüber hinaus kann es ebenfalls bevorzugt sein, daß erfindungsgemäße Verfahren an Lif-exprimierenden Zellen durchzuführen. Lysostaphin Immunity Factor (Lif) exprimierende Gram-positive Zellen zeigen Modifikationen im Mureingerüst der Zellwand. Diese Veränderungen haben keinen Einfluß auf die Verknüpfungsreaktionen von Zelloberflächenproteinen in der Zellwand. Das Ausführungsbeispiel 3 beschreibt eine bevorzugte Ausführungsform des erfindungsgemäßen Verfahrens zur Untersuchung der Verankerung von Proteinen in der Zellwand Lifexprimierender Zellen anhand der beispielhaft ausgewählten *S*. *hyicus* Lipase oder proLipFnBPB. Der Vergleich der enzymatischen Aktivität der Lipase an der Zellwand und im Überstand des Kulturmediums zeigte, daß die Lif-Expression keinen Einfluß auf die Sekretion der Lipase oder die Verankerung von prolipFnBPB in der Zelloberfläche hat.

In einer besonders bevorzugten Ausführungsform des erfindungsgemäßen Verfahrens erfolgt die Bestimmung der enzymatischen Aktivität der Reportersubstanz mittels Fluoreszenzspektroskopie, insbesondere konfokaler Fluoreszenzspektroskopie wie in Ausführungsbeispiel 3 gezeigt. Es ist hierbei möglich, auf die bekannten Verfahren der Ein- oder Mehrphotonenanregung zurückzugreifen. Als vorteilhafte Bestimmungsmethode hat sich insbesondere das Verfahren der Fluoreszenzkorrelationsspektroskopie (FCS) erwiesen, wie es in der WO 94/16313 detailliert beschrieben ist. Anstelle der in der vorgenannten Patentanmeldung beschriebenen Vorrichtung kann es auch bevorzugt sein, FCS unter Verwendung von Elementen aus der Nahfeldspektroskopie durchzuführen, wie in DE-C-44 38 391 und WO 96/13744 dargelegt. Auf die genannten Schriften wird ausdrücklich Bezug genommen zwecks Ergänzung der Beschreibung der vorliegenden Erfindung.

Die WO 98/16814, auf die hier zur Ergänzung der Beschreibung ausdrücklich Bezug genommen wird, beschreibt ein Verfahren zur Analyse von Partikel enthaltenden Proben durch wiederholte Messung der Photonenanzahl pro definiertem Zeitintervall von emittiertem oder in der Probe gestreutem Licht, gefolgt von der Bestimmung der Verteilungsfunktion der Photonenanzahl pro definiertem Zeitintervall, aus welcher sodann die Verteilungsfunktion der Partikelhelligkeiten bestimmt wird. Dieses Verfahren kann in bevorzugter Weise auch zur Untersuchung lumineszenter, insbesondere fluoreszierender, Proben eingesetzt werden und wird in dieser spezifischen Ausführungsform als sogenannte Fluoreszenzintensitätsverteilungsanalyse (fluorescence intensity distribution analysis, FIDA) bezeichnet. Auf den Offenbarungsgehalt dieser Anmeldungen wird ausdrücklich Bezug genommen.

Für Fluoreszenzmessungen geeignete Farbstoffe sind dem Fachmann aus der Literatur bekannt. Es kann z.B. bevorzugt sein, die Umsetzung eines Substrates, welches eine Änderung in seinen Fluoreszenzeigenschaften erfährt, zu bestimmen. Weiterhin kann es bevorzugt sein, einen Reporterassay unter Verwendung von fluoreszierenden bzw. luminogenen Proteinen wie GFP (Green Fluorescent Protein) einzusetzen.

Die genetische Veränderung gemäß Schritt b) des erfindungsgemäßen Verfahrens erfolgt in einer bevorzugten Ausführungsform mittels Transposon-Mutagenese. Stattdessen kann jedoch auch z.B. eine UV-Mutagenese oder eine chemische Mutagenese durchgeführt werden. Derartige Verfahren sind in zahlreichen Publikationen oder auch in Standard-Werken, auf die hier ausdrücklich zur Ergänzung der Beschreibung Bezug genommen wird, beschrieben (Miller J.H. (Ed): Experiments in Molecular Genetics, Cold Spring Harbor, New York 11724, Cold Spring Harbor Laboratory, 1977; Sambrock J., Fritsch E.F. und Maniatis T. (Eds): Molecular cloning, a laboratory manual; New York, Cold Spring Harbor Laboratory, 1989).

Der beispielhaft gewählte, nicht-pathogene Organismus *S. carnosus* zeigt eine ausgesprochen geringe Freisetzung von Zellwand-Untereinheiten und produziert keine störenden Exo-Proteasen. Seine Verwandtschaft zu den pathogenen Staphylococcen, wie *S*. *aureus, S. hyicus* oder *S. epidermidis,* ermöglicht den Transfer der hier beispielhaft erzielten Ergebnisse auf medizinisch relevantere Organismen.

So ist es beispielsweise möglich, das erfindungsgemäße Verfahren hinsichtlich der Schritte a) bis f) in dem nicht-pathogenen Organismus *S*. *carnosus* durchzuführen. Der gemäß Schritt f) identifizierte Abschnitt kann dann als Grundlage dienen, um nicht nur im Wildtyp-Stamm, sondern auch in verwandten Stämmen solche Nukleinsäuren zu identifizieren, die für einen Polypeptidfaktor kodieren, welcher die kovalente Verknüpfung von Polypeptiden an die Oberfläche dieser Stämme beeinflußt. Dies kann in einer dem Fachmann bekannten Weise erfolgen, wie z.B. mittels Hybridisierung mit Gensonden oder mittels PCR-Amplifikation der entsprechenden Gene.

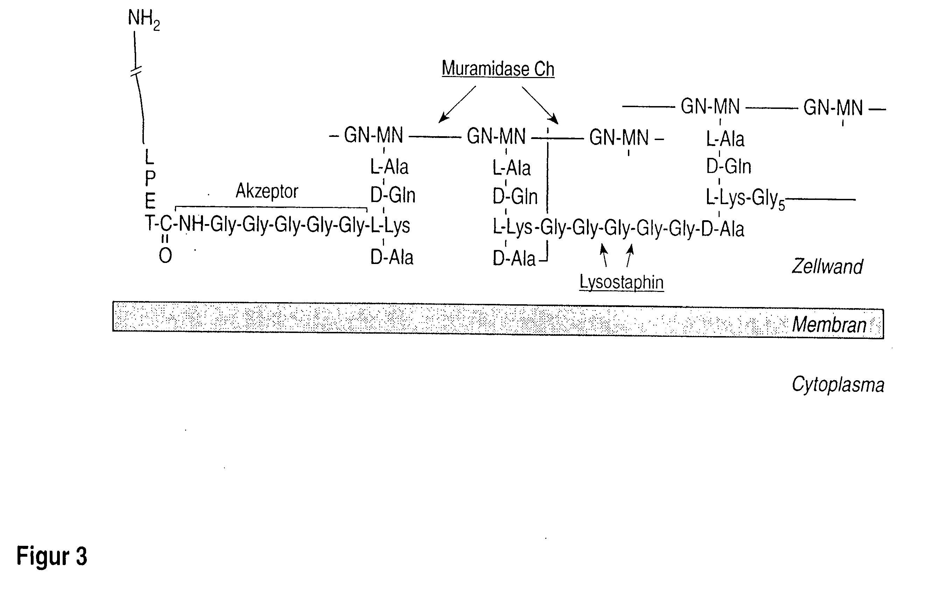

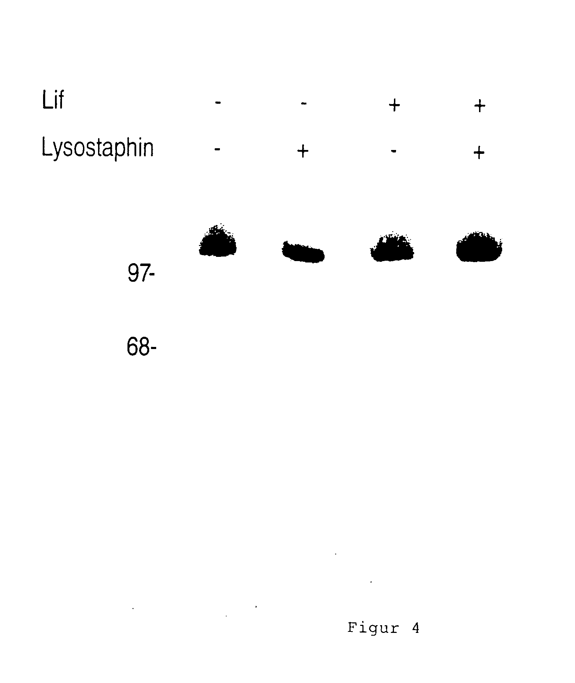

Eine weitere Ausführungsform des erfindungsgemäßen Verfahrens erfaßt neben der Bestimmung der enzymatischen Aktivität der Reportersubstanzen deren Verankerung an der Zelloberfläche. Diese im Ausführungsbeispiel 4 beschriebene weitere Form des erfindungsgemäßen Verfahrens ermöglicht es festzustellen, ob Proteine durch nicht-kovalente Bindungen an einer Freisetzung gehindert werden. Diese besondere Ausführungsform erlaubt es, zu bestimmen, auf welche Weise die Reportersubstanz an das Mureingerüst gebunden ist. Muramidase Ch schneidet vorzugsweise gemäß Figur 3 die Zellwand Gram-positiver Bakterien, so daß in der Zellwand verankerte Proteine mit Zellwandfragmenten variabler Länge abgespalten werden (Schneewind et al., EMBO J. 12: 4803-4811, 1993). Nicht-kovalent gebundene Proteine hingegen, die durch Muramidase Ch abgespalten werden, haben alle dieselbe Molmasse (Schneewind et al., EMBO J. 12: 4803-4811, 1993). Die Unterscheidung der verschiedenen Spaltprodukte erfolgt gemäß der besonderen Ausführungsform durch SDS-PAGE (Sodiumdodecylsulfat-Polyacrylamid-gelelektrophorese) und Immunoblotting. Beispielhaft ist das SDS-PAGE Laufverhalten von kovalent verankerten Proteinen nach Freisetzung durch Muramidase Ch und Lysostaphin-Behandlung in Figur 4 gezeigt.

In einer besonders bevorzugten Form des erfindungsgemäßen Verfahrens kann im Kulturmedium unterschieden werden, ob die Freisetzung von Zellwandproteinen durch Wirkung von genetischen Veränderungen, die den Mechanismus der Verankerung an der Oberfläche von Gram-positiven Bakterien beeinflussen oder durch natürliche Änderungen der Zellwand verursacht wurde. Zusätzlich zur erfindungsgemäßen Bestimmung der enzymatischen Aktivität erfolgt eine Charakterisierung der freigesetzten Polypeptide. Ausführungsbeispiel 5 zeigt dieses am Beispiel von proLipFnBPB aus pTX30 und pTX30/pCXlif exprimierenden Zellen. ProLipFnBPB, das durch natürliche Änderungen der Zellwand aus diesen Zellen freigesetzt wurde, zeigt bei der gelelektrophoretischen Untersuchung und anschließendem Immunoblotting nicht oder schlecht aufgelöste Banden (Schmier) aus Lipasen unterschiedlicher Länge. Direkt aus der Zelle freigesetzte Zellwandproteine zeigen, wie die durch Einwirkung von Lysostaphin (80 mg ml⁻¹ in BM, 30 min 37°C) gewonnenen Proteine aus dem Überstand von nicht-Lif exprimierenden Zellen, statt einer verschmierten aus einer Summe aus lipasespezifischen Signalen bestehenden Bande, eine schmale, stark begrenzte Bande bei der SDS-PAGE und anschließendem Immunoblotting.

Gegenstand der vorliegenden Erfindung sind die mittels des erfindungsgemäßen Verfahrens identifizierten und bevorzugt isolierten Nukleinsäuren.

Die angegebenen spezifischen Nukleinsäuresequenzen wurden durch Sequenzierung der an die Insertionsstellen der Transposons angrenzenden Bereich der genomischen *S*. *carnosus* erhalten.

Die Figur 1 beschreibt beispielhaft die Struktur eines als Reportersubstanz einsetzbaren Hybridproteins (Assay-Plasmid pTX30Δ82) sowie die oben erwähnten Strukturen der zur Simulation der erfindungsgemäßen Verfahren verwendeten Hybridproteine (Plasmide pTX30Δ82.sec und pTX30Δ82.mem) im Vergleich zur Struktur der *S. hyicus* Lipase (Plasmid pTX15). Am N-Terminus befindet sich das sog. Signalpeptid (schraffiert dargestellt), welches den Transport des Proenzyms (schematisch durch PP und Lipase dargestellt) durch die cytoplasmatische Membran ermöglicht und im Rahmen des sekretorischen Prozessierungsprozesses proteolytisch entfernt wird. In bevorzugter Weise schließt sich an die Lipase ein Linkerpeptid an, dessen Länge derart gewählt ist, daß die Lipase in einer inaktiven Konformation an der Oberfläche der Gram-positiven Bakterien kovalent verankert werden kann. Es folgen LPETG-Motiv, hydrophober und geladener Sequenzabschnitt (Assay-Plasmid pTX30Δ82). Das LPXTG-Motiv ist im zur Simulation verwendeten Hybridprotein (Plasmid pTx30Δ82.mem) durch die Sequenzabfolge ISQAS ersetzt. In dem zu einer weiteren Simulation verwendeten Hybridprotein (Plasmid pTX30Δ82.sec) schließt sich an das Linkerpeptid lediglich ein Sequen zabschnitt mit der Sequenz LPETGG an.

Figur 2 zeigt die Quantifizierung der Lipaseaktivität in den Simulations-Klonen. Die jeweilige Lipaseaktivität im Kulturüberstand wurde in Relation zur Gesamtaktivität des entsprechenden Klons bestimmt.

Figur 3 zeigt die Struktur des Peptidoglycans in Staphylococcen mit einem C-terminal verknüpften Oberflächenprotein. Die Schnit t-stellen für Muramidase Ch und Lysostaphin sind hervorgehoben.

In Figur 4 ist der Einfluß von Lysostaphin auf Zelloberflächenproteine, die durch Muramidase Ch aus der Zellwand von *S*. *carnosus* freigesetzt wurden, dargestellt. ProLipFnBPB wurde in Gegenwart (+) oder Abwesenheit (-) von Lif (pCXLif) in diesen Zellen synthetisiert. Die freigesetzten Hybridproteine wurden in Gegenwart (+) oder Abwesenheit (-) von Lysostaphin inkubiert und anschließend durch SDS-PAGE (10% Acrylamid) und Immunoblotting (Prolipase-spezifisches Antiserum) gemäß Strauß und Götz (Mol. Microbiol. 21:491-500, 1996) charakterisiert. Die Molmassen der Proteinstandards (in kDa) sind am linken Rand angegeben.

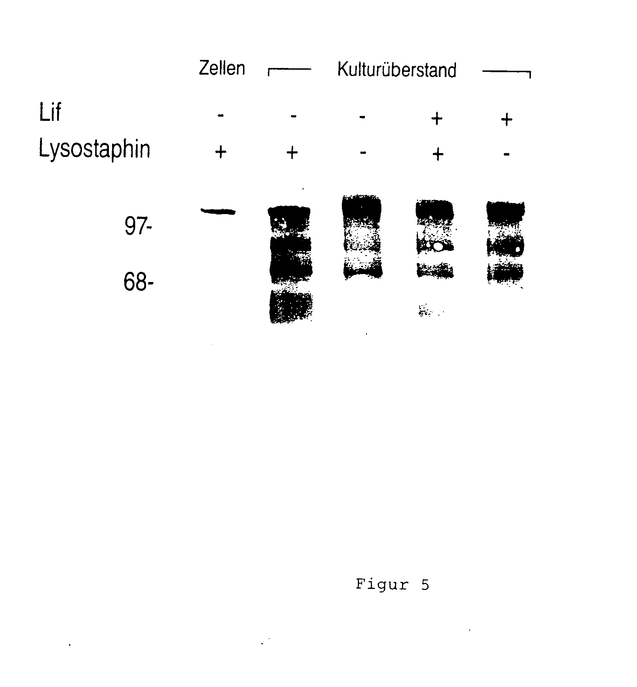

Figur 5 zeigt den Einfluß von Lysostaphin auf proLipFnBPB, das aus *S*. *carnosus* in das Kulturmedium durch natürliche Zellwandveränderungen freigesetzt wurde. Überstände von Zellen, die proLipFnBPB (pTX30) in Gegenwart (+) oder Abwesenheit (-) von Lif (pCXlif) exprimieren, wurden in Gegenwart (+) oder Abwesenheit (-) von Lysostaphin untersucht. Als Referenz diente proLipFnBPB, das durch Lysostaphineinwirkung aus der Zellwand von *S. carnosus* Zellen, die nur das Plasmid pTX30 enthalten, freigesetzt wurde. Die Proteine wurden durch SDS-PAGE (10% Acrylamid) aufgetrennt und Immunoblotting (Prolipase-spezifisches Antiserum) durchgeführt. Die Molmassen der Standardmoleküle sind am linken Rand angegeben.

### Ausführungsbeispiel 1

### Simulationsszenarien

### Stämme und Plasmide

Wildtyp-Stamm *S*. *carnosus* TM300 (Götz, F., J. Appl. Bacteriol. Symp. Supp. 69: 49-53, 1990) wurde als Wirtsorganismus für die Herstellung aller rekombinanter Staphylokokkenstämme benutzt. Die Herstellung des Assay-Plasmids pTX30Δ82 wird nachfolgend in Ausführungsbeispiel 2 beschrieben.

Die Plasmide pTX30Δ82.mem und pTX30Δ82.sec wurden analog zum Plasmid pTX30Δ82 unter Verwendung der Oligonukleotidpaare (Seq. IDNo. 12 bis 15) AS14(5'-ATAAGGCGCCTTAGTTTAATTATGCTTTGTGATTC)/AS45(5'-CGCAGGAAGCTTACCACAATCTAAGAAATCTGAAATATCTCAAGCAAGTGGAGA-AG) und AS42(5'-AATAAGGCGCCTCATTATCCACCTGTTTCAGGTAGTTC) / AS22(-5'-ACGAAAGCT TACCACAATCTAAGAAATCTGAAC) ausgehend von pTX30 hergestellt. Die Plasmide pTX30Δ82, pTX30Δ82.mem und pTX30Δ82.sec wurden in *S. carnosus* TM300 transformiert (Götz & Schumacher, FEMS Microbiol. Lett. 40: 285-288, 1987).

### Medien

Für die Kultivierung der Bakterien in Flüssigkultur wurde Basis-Medium (BM) verwendet, das 1% Pepton, 0.5% Hefe-Extrakt, 0.5% NaCl, 0.1% Glucose und 0.1% Di-Kaliumhydrogenphosphat enthielt (pH 7.4). Für die Induktion des Xylose Promoters wurde modifiziertes Basis-Medium (Induktionsmedium) benutzt, in dem die Glucose durch 0,5% Xylose ersetzt wurde. Nach Bedarf wurde das BM mit Chloramphenicol (Cm, 10 mg/l), Tetracyclin (Tc, 25 mg/l) und Erythromycin (Em, 2.5 mg/l) versetzt. Agar-Selektionsplatten enthielten zusätzlich 15 g/l Agar. Lipase-Testplatten wurden unter Verwendung von Tributyrin-Agar Basis (Merck) nach Anleitung des Herstellers zubereitet. Der Agar wurde vor dem Gießen der Platten mit 1% Glycerintributyrat und den entsprechenden Antibiotika (Tc, Em) versetzt. Auf diesen Platten können Lipasefreisetzende Bakterienzellen durch die Bildung klarer Höfe identifiziert werden.

### Part I:

### Lokalisation und Quantifizierung der Lipase-Aktivität in den Simulations-Klonen

Jeweils 5 ml Basismedium wurden von Platte mit Wildtyp-Stamm *S. carnosus* TM300 bzw. den oben beschriebenen *S*. *carnosus* Klonen beimpft und bei 37°C über Nacht unter Schütteln inkubiert. Mit diesen Vorkulturen wurden jeweils 5 ml Induktionsmedium 1:100 beimpft und bei 37°C bis zur spätlogarithmischen Wachstumsphase geschüttelt. Die Kulturen wurden auf 4°C gekühlt, bevor die Lipase-Aktivität im Kulturüberstand und die zellgebundene Lipase-Aktivität nach Freisetzung durch Lysostaphinbehandlung der Zellen gemäß Strauß und Götz (Mol. Microbiol. 21: 491-500, 1996) bestimmt wurde. Die Gesamtaktivität ergab sich aus der Summe der zellgebundenen Aktivität und der Aktivität im Kulturüberstand. Anschließend wurde die jeweilige Lipase-Aktivität im Kulturüberstand in Relation zur Gesamtaktivität des entsprechenden Klons bestimmt (Figur 2).

### Part II:

### Etablierung eines auf Fluoreszenzspektroskopie basierenden Assayverfahrens

Die *S. carnosus* Klone *S. carnosus*/pTX30Δ82, /pTX30Δ82.mem, und /pTX30Δ82.sec wurden über Nacht bei 37°C in Basis-Medium kultiviert. Die Zelldichten dieser Kulturen wurden durch Messung der optischen Dichten (OD576) im Photometer bestimmt und Verdünnungen (ca. 1:200) gleicher Zelldichte hergestellt. Für die Herstellung dieser Verdünnungen wurde modifiziertes Basis-Medium (Induktionsmedium) verwendet. Die verdünnten Kulturen ließ man daraufhin erneut bei 37°C herangewachsen. In verschiedenen Experimenten wurden sowohl Mikrotiterplatten als auch andere Gefäße für die Kultivierungen verwendet.

Zu unterschiedlichen Zeitpunkten der Kultivierung wurde die von den Bakterien freigesetzte Lipaseaktivität in den Kulturüberständen bestimmt. Die Zellen wurden durch Zentrifugation sedimentiert, die Kulturüberstände abgehoben und, wenn notwendig, auf Eis aufbewahrt. Assays wurden in Mikrotiterplatten (100 µl) mit Glasboden durchgeführt. Der Lipase-Assay-Puffer setzte sich wie folgt zusammen:
10 mM CaCl₂, 0,05% Triton-X-100, 20 mM Tris/HCl, pH 8,0. Als fluorogenes Farbsubstrat wurde 1,2-o-Dilauryl-rac-glycero-3-glutarsäure-resorufinester (Sigma # D7414) eingesetzt. Die Substrat-Stammlösung wurde in 100% DMSO bei 1 mg/ml angesetzt und bei -20°C gelagert. Pro Messprobe wurden jeweils 10 µl der Kulturüberstände mit 80 µl Lipase-Assay-Puffer und 10 µl Substrat-Lösung (10 µM Endkonzentration) vermischt. Die Umsetzung des Substrats wurde fluorometrisch unter Verwendung eines Fluoreszenz(ELISA)-Readers oder mittels eines Fluoreszenz-Korrelationsspektrometers (z.B. ConfoCor^{™}, Fa. Carl-Zeiss-Jena GmbH und Evotec, Deutschland) bestimmt.

Während der Klon *S*. *carnosus*/pTX30Δ82 in den Messungen nur unwesentlich höhere Signale als die Negativkontrolle (nur Puffer und Substrat) ergab, wurden in den Kulturüberständen der beiden Klone *S*. *carnosus*/pTX30Δ82. mem und *S. carnosus*/pTX30Δ82. sec signifikante Mengen an Lipaseaktivität gefunden.

### Ausführungsbeispiel 2

### Screening von Mutanten mit Defekten in der Oberflächenverankerung infolge einer Transposon-Mutagenese

### Stämme und Plasmide

Wildtyp-Stamm *S*. *carnosus* TM300 (Götz, F., J. Appl. Bacteriol. Symp. Supp. 69: 49-53, 1990) wurde wie von Götz und Schumacher (FEMS Microbiol. Lett. 40: 285-288, 1987) beschrieben mit dem Assay-Plasmid pTX30Δ82 transformiert. Plasmid pTX30Δ82 wurde analog zum Plasmid pCX30Δ82 (Strauß und Götz, Mol. Microbiol. 21:491-500, 1996) hergestellt. Es diente jedoch nicht das mit Chloramphenicol selektionierbare Plasmid pCX15 (Wieland et al., Gene 158: 91-96, 1995), sondern das mit Tetracyclin selektionierbare Plasmid pTX15 (Peschel et al., FEMS Microbiol. Lett. 137: 279-284, 1996) als Ausgangsvektor. Dieses Plasmid enthält eine Genfusion, welche das Assay-Hybridprotein (proLipFnBPBΔ82) bestehend aus *S. hyicus* Lipase und dem C-terminalen Teil des Fibronectin Bindeprotein B (FnBPB) kodiert und welche unter der Kontrolle des induzierbaren Xylose-Promotors steht (Wieland et al., Gene 158: 91-96, 1995). Der Abstand zwischen dem C-terminalen Alanin-Rest der Lipase und dem Leucin-Rest des LPXTG-Motives des FnBPB betrug 10 Aminosäuren (Figur 1). Für die Transposon-Mutagenese wurde dieser Stamm zusätzlich mit dem Plasmid pTV1ts (Youngman et al. in: Smith et al. (ed): Regulation of procaryotic development. American Society for Microbiology, Washington DC, 65-87, 1989) transformiert.
Die Sequenzen der hierin verwendeten Nukleinsäuren sind wie folgt in Genbanken abgelegt: Genbank-Entry-Number: Plasmid pT181: g151679; *xyl*R (*S. xylosus*): g48833; *lip* (*S. hyicus*): g488333; Plasmid pC194: g150548; *fnb*B (*S. aureus*): g49040.

Die hier genannten Literaturzitate sind die Beschreibung ergänzend heranzuziehen.

### Medien

Es wurden Medien entsprechend der Angaben in Ausführungsbeispiel 1 verwendet.

### Transposon-Mutagenese

Das Plasmid pTV1ts, welches in Gram-positiven Bakterien eine temperatur-sensitive Replikation zeigt, kodiert für eine Chloramphenicol-Resistenz und trägt das Transposon Tn917, das ein Erythromycin-Resistenzgen.beinhaltet (Youngman et al. in: Smith et al. (ed.): Regulation of procaryotic development. American Society for Microbiology, Washington DC, 65-87, 1989). *S. carno*sus Zellen, welche die Plasmide pTV1ts und pTX30Δ82 trugen, wurden über Nacht in BM-Selektionsmedium (Cm, Tc, Em) bei 30°C angezogen. Mit dieser Vorkultur wurde BM-Selektionsmedium (Tc, Em) 1:100 inocculiert, und das Tn917 bei der nicht-permissiven Temperatur von 40°C für mindestens 20 Zellgenerationen mobilisiert.

### Screening von Mutanten mit Defekten in der Oberflächenverankerung

Mutanten mit einer Transposon-Mobilisierungsrate (Zellen Tc+Em resistent, aber Cm sensitiv) von mindestens 1000:1 wurden direkt auf Lipase-Testplatten ausplattiert und bei 30°C für ca. 48 h bebrütet, bevor potentielle Mutanten mit Defekten in der kovalenten Zellwand-Verankerung identifiziert wurden. Diese zeichneten sich durch einen durch die freigesetzte Lipase-Aktivität erzeugten klaren Hof aus. Auf Agar-Selektionsplatten (Tc, Em) wurden die relevanten Klone nochmals vereinzelt, bevor ihr Phänotyp ein weiteres Mal auf Lipase-Testplatten und in Flüssigkultur verifiziert wurde. Hierzu wurden jeweils 5 ml InduktionsMedium von Platte beimpft und bei 30°C über Nacht geschüttelt, bevor die Lipaseaktivität im Kulturüberstand im Vergleich zum nicht-mutierten Ausgangsstamm, wie von Strauß und Götz (Mol. Microbiol. 21: 491-500, 1996) beschrieben, bestimmt wurde.

### Ausführungsbeispiel 3:

### Stämme und Plasmide

Der Wildtyp-Stamm S. *carnosus* TM300 (Götz, F., J. Appl. Bacteriol. Symp. Supp. 69: 49-53, 1990) wurde als Wirtsorganismus für die Herstellung aller rekombinater Staphylococcenstämme benutzt. Die Plasmide pTX15, pTX30 (kodiert ProLipFnBPB, ein Hybridprotein bestehend aus *S. hyicus* Lipase verbunden mit dem C-Terminus des S. *aureus* Fibronectin Bindungsproteins B) und pCXlif (lif, lysostaphin immunity factor) wurden in *S. carnosus* TM 300, wie von Götz und Schuhmacher (FEMS, Microbiol. Lett. 40:285-288, 1987) beschrieben, transformiert.

Die Konstruktion von pCXlif erfolgte gemäß des von Thumm und Götz (Mol. Microbiol. 23:1251-1256, 1997) beschriebenen Verfahrens.

Das Plasmid pTX30 wurde durch Insertion, des *Bam*HI-*Nar*I Fragmentes von pCX30 (Strauß und Götz, Mol. Microbiol. 21:491-500, 1996) in das mit denselben Restriktionsenzymen geschnittene Plasmid pTX15 eingefügt.

Die Gene wurden unter Kontrolle des Xylose-Promotor-Systems exprimiert (Wieland et al., Gene 158:91-96,1995). Die Induktion der Expression erfolgte wie bei Strauß und Götz (Mol. Microbiol. 21:491-500, 1996) beschrieben.

Die genannten Literaturzitate sind die Beschreibung ergänzend heranzuziehen.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

*Der Einfluß des Lysostaphin immunity factors (Lif) auf die Sekretion und die Verankerung von S. hyicus Lipase oder proLipFnBPB in der Zellwand durch vergleich der Lipaseaktivität an der Zellwand und im Überstand des Kulturmediums.*

Die Zellkulturen (pTX15, pTX30, pTX15+pCXlif, pTX30+pCXlif) wurden zunächst durch Zentrifugation in Zellpellets und Medium getrennt. Anschließend wurden die Pellets dreimal mit BM gewaschen und in BM aufgenommen. Als Referenz dienten Zellwandproteine, die durch Behandlung mit Lysostaphin (80 µg/ml in BM; 30 min bei 37°C) von pTX30 exprimierenden Zellen freigesetzt wurden. Von den Proben wurden Verdünnungen angefertigt. Hierzu wurden jeweils 5 µl der Kulturüberstände mit 95 µl Lipase-Assay-Puffer (10mM CaCl₂, 0.1% Triton X-100 und 20 mM Tris-HCl, pH 8.5), der das chromogene Lipasesubstrat p-Nitrophenyl Caprylat [Sigma] in einer Konzentration von 5 mM enthielt, versetzt. Die Hydrolyse des Substrates wurde anschließend über 10 Minuten bei 30°C photometrisch unter Verwendung eines Mikrotiterplatten (Elisa)-Readers (SpectraMax, Molecular Devices) oder mittels Fluoreszenz-Korrelationsspektroskopie mit dem ConfoCor bei einer Wellenlänge von 405 nm verfolgt. Die Assays wurden in Mikrotiterplatten ohne bzw. mit Glasboden durchgeführt.

Insgesamt zeigte sich, daß in Zellen, die pXT15 exprimieren die Gesamtlipaseaktivität im Überstand 99.2% und in Zellen die sowohl pXT15 als auch pCXlif exprimieren 99.1% beträgt. Im Vergleich dazu zeigten Zellen die pTX30 enthalten 85.1% ihrer Lipaseaktivität an der Zelloberfläche, wie auch Zellen, die sowohl pTX30 als auch pCXlif exprimieren (84.5%)

Es konnte somit gezeigt werden, daß die Lif Expression keinen Einfluß auf die Sekretion der Lipase oder die Verankerung von prolipFnBPB in der Zelloberfläche hat.

### Ausführungsbeispiel 4

### Stämme und Plasmide

Die verwendeten Stämme und Plasmide entsprechen den in Ausführungsbeispiel 2 genannten.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

### Bestimmung der enzymatischen Aktivität der freigesetzten Proteine

Es wurde die von den Bakterien freigesetzte Lipaseaktivität in den Kulturüberständen wie folgt bestimmt:

Die Zellen wurden durch Zentrifugation sedimentiert, die Kulturüberstände abgehoben und, wenn notwendig, auf Eis aufbewahrt. Assays wurden in Mikrotiterplatten ohne Glasboden durchgeführt. Der Lipase-Assay-Puffer (10mM CaCl₂, 0.1% Triton X-100 und 20 mM Tris-HCl, pH 8.5), enthielt das chromogene Lipasesubstrat p-Nitrophenyl Caprylat [Sigma] in einer Konzentration von 5 mM. Pro Messprobe wurden jeweils 5 µl der Kulturüberstände mit 95 µl Lipase-Assay-Puffer vermischt. Die Umsetzung des Substrats wurde photometrisch unter Verwendung eines Mikrotiterplatten (ELISA)-Readers bestimmt.

### Unterscheidung zwischen kovalent und nicht-kovalent an die Zellwand gebundenen Proteinen

Um auszuschließen, daß proLipFnBPB nicht-kovalent an die Zellwand der exprimierenden Zellen gebunden ist und aus diesem Grund bei der Bestimmung der enzymatischen Aktivität im Überstand nicht erfaßt wird, wurde eine Strategie entwickelt, die auf der Verwendung von Muramidase Ch und Lysostaphin basiert. Muramidase Ch hydrolysiert die β-1,4 Bindung von N-Acetylmuraminsäure und Acetylglucosamin (Figur 3) (Ghuysen, Bacteriol. Rev. 32: 425-464, 1968).
Es schneidet nicht direkt an den Verknüpfungspunkten der Oberflächenproteine mit der Zellwand, so daß die Proteine mit Zellwandfragmenten variabler Länge abgespalten werden (Schneewind et al., EMBO J. 12: 4803-4811, 1993).
Nicht-kovalent gebundene Proteine hingegen, die durch Muramidase Ch abgespalten werden, haben alle dieselbe molekulare Masse (Schneewind et al., EMBO J. 12: 4803-4811, 1993).

Die genannten Literaturzitate sind die Beschreibung ergänzend heranzuziehen.

Die Zellen wurden aus 500 µl Kulturmedium durch Zentrifugation gewonnen. Anschließend wurden die Pellets dann drei mal mit Wasser gewaschen und durch Zugabe von Trichloressäure (7% wt/Vol) ausgefällt (20 min auf Eis). Nach der Zentrifugation des Präzipitates wurde das entstehende Pellet zweimal mit Azeton gewaschen und im Vakuum getrocknet. Die Pellets wurden dann in 170 µl BM, dem Muramidase Ch (100 µg ml⁻¹) zugesetzt wurde, gelöst und die Lösung für 3h bei 37°C inkubiert. Anschließend wurden die Proben erneut zentrifugiert und der Überstand in zwei Aliquots von je 80 µl aufgeteilt, wovon eins mit 20 µl Wasser, das andere mit 20 µl Lysostaphin-Lösung (400 mg ml⁻¹) versetzt wurde. Die Lösungen wurden für 30 min bei 37°C inkubiert. Die einzelnen Aliquots wurden nun aufkonzentriert und unter Anwendung von SDS-PAGE (10% Acrylamid) und Immunoblotting (mit Prolipase spezifischem Antiserum) untersucht.

Es zeigte sich (Figur 4), daß Muramidase Ch zur vollständigen Freisetzung von proLipFnBPB aus der Zellwand von *S*. *carnosus* führt, unabhängig davon ob pCXlif in diesen Zellen ebenfalls exprimiert wurde. In beiden Fällen war auf dem Gel ein Spektrum von Lipase-spezifischen Signalen als Schmier zu erkennen, der auf kovalent an proLipFnBPB gebundene Zellwandfragmente unterschiedlicher Länge zurückzuführen ist.

Zur Unterscheidung von Lif exprimierenden Zellen wurden die Proben in einer parallel durchgeführten Untersuchung vor Durchführung der Gelelektrophorese und des Immunoblottings mit Lysostaphin behandelt. Es zeigt sich, daß in den Fällen in denen proLipFnBPB aus Zellen, die kein Lif exprimieren, freigesetzt wurde, die Reste der Zellwandverankerung vollständig entfernt werden. Auf dem Gel war dies als exakte Bande zu erkennen. Oberflächenproteine, die aus Lif exprimierenden Zellen stammten, waren nicht Lysostaphin sensitiv (Figur 4).

### Ausführungsbeispiel 5

### Stämme und Plasmide

Die verwendeten Stämme und Plasmide entsprechen den in Ausführungsbeispiel 2 genannten.

### Medien

Die Bakterien wurden in Basis-Medium (BM; siehe Ausführungsbeispiel 1) bei 30°C kultiviert. Nach Bedarf wurde das Basismedium mit Chloramphenicol (Cm, 10mg/l) oder Tetracyclin (Tc, 25 mg/l) versetzt.

### Bestimmung der enzymatischen Aktivität der freigesetzten Proteine

Es wurde die von den Bakterien freigesetzte Lipaseaktivität in den Kulturüberständen gemäß Ausführungsbeispiel 3 bestimmt.

*Bestimmung des Anteils durch natürliche Zellwandveränderungen freigesetzter Oberflächenproteine aus S. carnosus.*

Ein wichtiges Charakteristikum durch natürliche Zellwandveränderungen freigesetzter Zellwandproteine ist, daß sie vor der Freisetzung kovalent an die Zellwand gebunden sind.

Zur Bestimmung des Anteils natürlich freigesetzter Proteine wurde der Kulturüberstand von Zellen, die die Plasmide pCXlif und/oder pTX30 enthalten, aufkonzentriert und durch SDS-PAGE und Immunoblotting analysiert. Als Referenz diente proLipFnBPB das durch Lysostaphin aus Zellen, die nur das Plasmid pTX30 enthalten, freigesetzt wurde. Es wurden zusätzlich zu Zersetzungsprodukten, die eine größere elektrophoretische Beweglichkeit als die Referenz besaßen, eine Summe von Lipase spezifischen Signalen, als Schmier beobachtet. Inkubation des Überstandes mit Lysostaphin (80 mg ml⁻¹ in BM, 30 min 37°C) vor Gelelektrophorese und Immunoblotting hatte nur einen Effekt auf Proteine, die aus dem Überstand von nicht-Lif exprimierenden Zellen gewonnen wurden. Statt dem Schmier aus einer Summe aus lipase-spezifischen Signalen ist eine begrenzte Bande zu sehen, die dieselbe elektrophoretische Beweglichkeit wie die Referenz zeigt. Die lipasespezifischen Signale, die aus Zellen, die Lif und proLipFnBPB exprimieren stammen, werden nicht durch Lysostaphin beeinflußt.

In dieser Untersuchung zeigte sich darüber hinaus, daß durch natürliche Freisetzung insgesamt 5% der Gesamtlipaseaktivität von Zellen, die ProLipFnBPB durch low-copy-number Plasmide exprimieren im Überstand gemessen wurde. Wohingegen bei mediumcopy-number Plasmiden sogar 15% der Gesamtlipaseaktivität im Überstand zu bestimmen war. Die Koexpression anderer Oberflächenproteine hatte keinen Einfluß auf die Freisetzung der Lipase.

## Patentansprüche

1. Verfahren zum Identifizieren einer Nukleinsäure, die für einen Polypeptidfaktor kodiert, welcher die kovalente Verknüpfung von Polypeptiden an die Oberfläche von Gram-positiven Bakterien beeinflußt, mit folgenden Schritten:
a) Bereitstellen einer Probe Gram-positiver Bakterien, welche genetisch veränderbar sind und mindestens eine kovalent mit der Oberfläche der Gram-positiven Bakterien verbundene oder verbindbare enzymatische Reportersubstanz aufweisen oder bilden, wobei die mindestens eine Reportersubstanz ohne kovalente Bindung an die Oberfläche der Gram-positiven Bakterien eine andere enzymatische Aktivität aufweist als bei kovalenter Bindung an die Oberfläche der Gram-positiven Bakterien,
b) Verursachen von genetischen Veränderungen in Gram-positiven Bakterien der Probe,
c) Bestimmen der enzymatischen Aktivität der Reportersubstanz der Gram-positiven Bakterien der Probe,
d) Separieren von Gram-positiven Bakterien, die eine andere enzymatische Aktivität der Reportersubstanz aufweisen als die, die bei kovalenter Bindung der Reportersubstanz an die Oberfläche der Gram-positiven Bakterien auftritt,
e) Isolieren der Nukleinsäure der in Schritt d) separierten Gram-positiven Bakterien,
f) Identifizieren mindestens eines Abschnittes der in Schritt e) isolierten Nukleinsäure, der die genetische Veränderung trägt,
g) Identifizieren einer Nukleinsäure, die für einen Polypeptidfaktor kodiert, welcher die kovalente Verknüpfung, von Polypeptiden an die Oberfläche von Gram-positiven Bakterien beeinflußt, auf der Grundlage des in Schritt f) identifizierten Abschnittes.

2. Verfahren nach Anspruch 1, **dadurch gekennzeichnet, daß** die enzymatische Aktivität der Reportersubstanz im Vergleich zu mindestens einer Referenzprobe, die nicht genetisch verändert wurde, und/oder mindestens einer Referenzprobe, in der die Reportersubstanz nicht-kovalent an die Oberfläche Gram-positiver Bakterien gebunden ist, und/oder mindestens einer Referenzprobe, in der die Reportersubstanz kovalent an die Oberfläche der Gram-positiven Bakterien gebunden ist, bestimmt wird.

3. Verfahren nach einem der Ansprüche 1 und/oder 2, **dadurch gekennzeichnet, daß** die gemäß Schritt 1g), identifizierte Nukleinsäure gewonnen wird. ,

4. Verfahren nach mindestens einem der Ansprüche 1 bis 3, **dadurch gekennzeichnet, daß** die kovalente Verknüpfung der Polypeptide an das Murein der Zellwand, insbesondere an Interpeptidbrücken, wie z.B. Pentaglycine, Gram-positiver Bakterien erfolgt.

5. Verfahren nach mindestens einem der Ansprüche 1 bis 4, **dadurch gekennzeichnet, daß** die Polypeptide Pathogenitätsfaktoren Gram-positiver Bakterien darstellen.

6. Verfahren nach mindestens einem der Ansprüche 1 bis 5,
**dadurch gekennzeichnet, daß** die Reportersubstanz ein Hybridpolypeptid ist.

7. Verfahren nach Anspruch 6, **dadurch gekennzeichnet, daß** das Hybridpolypeptid eine Anordnung der folgenden Sequenzabschnitte aufweist: N-terminales Signalpeptid, Enzym, Sequenzabschnitt mit der Sequenz LPXTG, hydrophober Sequenzabschnitt und geladener Sequenzabschnitt.

8. Verfahren nach Anspruch 7, **dadurch gekennzeichnet, daß** das Enzym als Proenzym bereitgestellt wird.

9. Verfahren nach einem der Ansprüche 6 bis 8, **dadurch gekennzeichnet, daß** die Änderung der enzymatischen Aktivität durch einen Übergang des Enzyms aus einer inaktiven in eine aktive Konformation oder umgekehrt bedingt ist.

10. Verfahren nach mindestens einem der Ansprüche 6 bis 9 **dadurch gekennzeichnet, daß** zwischen Enzym und Sequenzabschnitt mit der Sequenz LPXTG ein Linkerpeptid, welches insbesondere weniger als 10 Aminosäuren umfaßt, angeordnet ist.

11. Verfahren nach mindestens einem der Ansprüche 1 bis 10, **dadurch gekennzeichnet, daß** die Bestimmung der enzymatischen Aktivität der Reportersubstanz mittels Fluoreszenzspektroskopie, insbesondere konfokaler Fluoreszenzspektroskopie erfolgt.

12. Verfahren nach mindestens einem der Ansprüche 1 bis 11, **dadurch gekennzeichnet, daß** die Gram-positiven Bakterien Lysostaphin Immunity Factor (Lif) exprimieren.

13. Verfahren nach mindestens einem der Ansprüche 1 bis 12, **dadurch gekennzeichnet, daß** der Anteil der durch natürliche Zellwandveränderungen freigesetzten Reportersubstanzen bestimmt wird.

14. Verfahren nach mindestens einem der Ansprüche 1 bis 13, **dadurch gekennzeichnet, daß** der Anteil nicht-kovalent mit der Oberfläche Gram-positiver Bakterien verbundener Reportersubstanzen bestimmt wird.

## Claims

1. Process for identifying a nucleic acid, which codes for a polypeptide factor, which influences the covalent linking of polypeptides to the surface of Gram-positive bacteria, having the following steps:
a) providing a sample of Gram-positive bacteria which can be modified genetically and have or form at least one enzymatic reporter substance bound or which can be bound covalently to the surface of Gram-positive bacteria, wherein the at least one reporter substance without covalent binding to the surface of Gram-positive bacteria has a different enzymatic activity than for covalent binding to the surface of Gram-positive bacteria,
b) causing genetic changes in Gram-positive bacteria of the sample,
c) determining the enzymatic activity of the reporter substance of Gram-positive bacteria of the sample,
d) separating Gram-positive bacteria, which have a different enzymatic activity of the reporter substance than that which occurs for covalent binding of the reporter substance to the surface of Gram-positive bacteria,
e) isolating the nucleic acid of Gram-positive bacteria separated in step d),
f) identifying at least one section of the nucleic acid isolated in step e), which carries the genetic modification,
g) identifying a nucleic acid, which codes for a polypeptide factor, which influences the covalent linking of polypeptides to the surface of Gram-positive bacteria, on the basis of the section identified in step f).

2. Process according to claim 1, **characterised in that** the enzymatic activity of the reporter substance is determined compared to at least one reference sample, which has not been modified genetically, and/or at least one reference sample, in which the reporter substance is not bound covalently to the surface of Gram-positive bacteria, and/or at least one reference sample, in which the reporter substance is bound covalently to the surface of Gram-positive bacteria.

3. Process according to one of claims 1 and/or 2, **characterised in that** the nucleic acid identified according to step 1g) is recovered.

4. Process according to at least one of claims 1 to 3, **characterised in that** the covalent linking of polypeptides takes place on the murein of the cell wall, in particular on interpeptide bridges, such as for example pentaglycines, of Gram-positive bacteria.

5. Process according to at least one of claims 1 to 4, **characterised in that** the polypeptides represent pathogenicity factors of Gram-positive bacteria.

6. Process according to at least one of claims 1 to 5, **characterised in that** the reporter substance is a hybrid polypeptide.

7. Process according to claim 6, **characterised in that** the hybrid polypeptide has an arrangement of the following sequence sections: N-terminal signal peptide, enzyme, sequence section with the sequence LPXTG, hydrophobic sequence section and charged sequence section.

8. Process according to claim 7, **characterised in that** the enzyme is provided as a proenzyme.

9. Process according to one of claims 6 to 8, **characterised in that** the change in enzymatic activity is caused by a transition of the enzyme from an inactive to an active conformation or vice versa.

10. Process according to at least one of claims 6 to 9, **characterised in that** a linker peptide, which comprises in particular fewer than 10 amino acids, is arranged between enzyme and sequence section having the sequence LPXTG.

11. Process according to at least one of claims 1 to 10, **characterised in that** the determination of the enzymatic activity of the reporter substance takes place by means of fluorescence spectroscopy, in particular confocal fluorescence spectroscopy.

12. Process according to at least one of claims 1 to 11, **characterised in that** the Gram-positive bacteria express Lysostaphin Immunity Factor (Lif).

13. Process according to at least one of claims 1 to 12, **characterised in that** the proportion of reporter substances released by natural changes in the cell wall is determined.

14. Process according to at least one of claims 1 to 13, **characterised in that** the proportion of reporter substances not bound covalently to the surface of Gram-positive bacteria is determined.

## Revendications

1. Procédé d'identification d'un acide nucléique, qui code un facteur polypeptidique, lequel influence la liaison covalente de polypeptides sur la surface de bactéries Gram-positives, avec les étapes suivantes :
a) préparation d'un échantillon de bactéries Gram-positives, qui sont génétiquement modifiables et présentent ou forment au moins une substance reporter enzymatique liée ou liable à la surface de bactéries Gram-positives, où la au moins une substance reporter présente sans liaison covalente sur la surface de bactéries Gram-positives, une autre activité enzymatique que lors de la liaison covalente sur la surface de bactéries Gram-positives,
b) induction de modifications génétiques dans les bactéries Gram-positives de l'échantillon,
c) détermination de l'activité enzymatique de la substance reporter des bactéries Gram-positives de l'échantillon,
d) séparation des bactéries Gram-positives, qui présentent une autre activité enzymatique de la substance reporter que celle qui se produit lors de la liaison covalente de la substance reporter sur la surface de bactéries Gram-positives,
e) isolement de l'acide nucléique des bactéries Gram-positives séparées à l'étape d),
f) identification d'au moins un segment de l'acide nucléique isolé à l'étape e), qui porte la modification génétique,
g) identification d'un acide nucléique, qui code un facteur polypeptidique, qui influence la liaison covalente de polypeptides sur la surface de bactéries Gram-positives, sur base du segment identifié à l'étape f).

2. Procédé selon la revendication 1, **caractérisé en ce que** l'activité enzymatique de la substance reporter est déterminée par comparaison à au moins un échantillon de référence, qui n'a pas été modifié génétiquement, et/ou au moins un échantillon de référence, dans lequel la substance reporter est liée de manière non covalente à la surface de bactéries Gram-positives, et/ou au moins un échantillon de référence, dans lequel la substance reporter est liée de manière covalente à la surface de bactéries Gram-positives.

3. Procédé selon la revendication 1 ou 2, **caractérisé en ce que** l'on obtient l'acide nucléique identifié selon l'étape 1g).

4. Procédé selon au moins une des revendications 1 à 3, **caractérisé en ce que** la liaison covalente du polypeptide est réalisée sur la muréïne de paroi cellulaire, en particulier sur des ponts interpeptidiques, comme par exemple pentaglycine, de bactéries Gram-positives.

5. Procédé selon au moins une des revendications 1 à 4, **caractérisé en ce que** les polypeptides représentent des facteurs pathogènes de bactéries Gram-positives.

6. Procédé selon au moins une des revendications 1 à 5, **caractérisé en ce que** la substance reporter est un polypeptide hybride.

7. Procédé selon la revendication 7, **caractérisé en ce que** le polypeptide hybride présente une disposition des segments suivants de séquence : peptide signal N-terminal, enzyme, segment de séquence avec la séquence LPXTG, segment de séquence hydrophobe et segment de séquence chargé.

8. Procédé selon la revendication 7, **caractérisé en ce que** l'enzyme est préparé comme une pro-enzyme.

9. Procédé selon l'une des revendications 6 à 8, **caractérisé en ce que** la modification de l'activité enzymatique est conditionnée par un transfert de l'enzyme depuis une conformation inactive en une conformation active ou inversement.

10. Procédé selon au moins une des revendications 6 à 9, **caractérisé en ce qu'**entre l'enzyme et le segment de séquence avec la séquence LPXTG, est disposé un peptide lieur, qui comprend en particulier, moins de 10 acides aminés.

11. Procédé selon au moins une des revendications 1 à 10, **caractérisé en ce que** la détermination de l'activité enzymatique de la substance reporter est réalisée par la spectroscopie à fluorescence, en particulier la spectroscopie confocale à fluorescence.

12. Procédé selon au moins une des revendications 1 à 11, **caractérisé en ce que** les bactéries Gram-positives expriment le facteur d'immunité lysostaphine (Lif).

13. Procédé selon au moins une des revendications 1 à 12, **caractérisé en ce que** la quantité de substances récepteur libérées par les modifications naturelles de membrane cellulaire est déterminée.

14. Procédé selon au moins une des revendications 1 à 13, **caractérisé en ce que** la quantité de substance reporter liée de manière non covalente à la surface de bactéries Gram-positives est déterminée.
